# EUROPEAN PATENT SPECIFICATION

(11) **EP 2 249 742 B1**
(45) Date of publication and mention of the grant of the patent: **21.06.2017**
(21) Application number: 09711088.6
(22) Date of filing: 13.02.2009
(51) Int. Cl.: A61F 2/06

(54) **TISSUE ENGINEERING SCAFFOLDS**
GEWEBEZÜCHTUNGSGERÜSTE
ÉCHAFAUDAGES DE GÉNIE TISSULAIRE

(30) Priority: 14.02.2008 US 28860 P
(43) Date of publication of application: 17.11.2010
(73) Proprietor: RegenMed (Cayman) Ltd., George Town Grand Cayman KY1-1104 (KY)
(72) Inventor: RAPOPORT, H., Scott, 48010 Bilbao, Bizkaia (ES); FISH, Jeffrey, E., Winston-Salem NC 27127 (US); ILAGAN, Roger, M, Burlington, NC 27215 (US); NAMTATA, Sangha, Winston-Salem, NC 27104 (US); ROBBINS, Neil, F Jr., Winston-Salem, NC 27103 (US); GUTHRIE; Kelly, I, Winston-Salem, NC 27104 (US); PAYNE, Richard, Winston-Salem, NC 27103 (US); JAIN, Deepak, Winston-Salem, NC 27103 (US)
(74) Representative: Denison, Christopher Marcus
(86) International application number: PCT/US2009/034137
(87) International publication number: WO 2009/103012

(56) References cited:
- EP-A1- 0 157 178
- WO-A1-96/00103
- WO-A2-2004/096095
- US-A- 5 282 847
- US-A1- 2003 211 130
- US-A1- 2005 119 733
- US-A1- 2005 251 249
- US-A1- 2007 298 072

## Description

### Field of the Invention

The present invention relates to tissue engineering scaffolds that mimic the biomechanical behavior of native blood vessels, and methods of making and using the same.

### Background of the Invention

A major problem in blood vessel tissue engineering is the construction of vessel grafts that possess suitable, long-lasting biomechanical properties commensurate with native vessels. Arterial replacements pose special challenges due to both the cyclic loading common to all vessels, but additionally the higher operating pressure required of those vessels. Researchers have approached this problem through a variety of synthetic and organic materials, different construction modalities (e.g. electrospinning and casting) and numerous composite designs. For example, attempts have been made to create blood vessel grafts using various combinations of donor grafts, natural components, and synthetic components (see e.g. Zilla et al., U.S. Published Patent Application 2005/0131520; Flugelman, U.S. Published Patent Application 2007/0190037; Shimizu, U.S. Patent 6,136,024; Matsuda et al., U.S. Patent 5,718,723; and Rhee et al., U.S. Patent 5,292,802). Other scaffolds composed of poly (ester urethane) ureas (PEUU) (Courtney et al. (2006) Biomaterials. 27:3631-3638), and PEUU/collagen (Guan et al. (2006) Cell Transplant. Vol. 15. Supp. 1;S17-S27) have been reported as exhibiting tissue-like functional properties. However, although synthetic materials such as Dacron® (ethylene terephthalate) and PTFE (Teflon) have been successfully used for large diameter vessels, no synthetic material has been successfully utilized for small diameter (e.g. less than 6 mm internal diameter) vascular grafts. Vascular grafts composed of Dacron® (ethylene terephthalate) and PTFE having an internal diameter of less than 5 mm have been found to be clinically unacceptable due to acute thrombus formation and chronic anastomotic and/or intimal hyperplasia (Walpoth et al. (2005) Expert Rev. Med. Dev. 2(6):647-51). The elusive success of small-diameter vascular grafts might be in part attributable to factors including the failure to properly match *in vivo* mechanical properties.

The biomechanical properties of native blood vessels have been extensively characterized. It has become apparent that their response to stress and strain is an important feature (Roach et al. (1957) Can. J. Biochem. Physiol. 35:681-690; Gosline & Shadwick (1998) American Scientist. 86:535-541). Materials that exhibit a stress-strain curve known as a "J-shaped" curve are candidates that may be suitable for use in a tissue engineering scaffold, such as a blood vessel scaffold, wherein a mechanical response to stress and strain resembling that of a native blood vessel is desirable. The mechanical properties of various fabricated scaffolds made from blends of elastin, collagen, and synthetic polymers have been reported (Lee et al. (2007) J. Biomed. Mater. Res. A., Dec 15;83(4):999-1008; Smith et al. (2008) Acta Biomater. Jan;4(1):58-66; Lelkes et al. U.S. Published App. No. 2006/0263417). EP 0 157 178 A1 describes an artificial vessel having a compliance and a stress-strain curve approximate to those of a vital vessel. The vessel has a porous part and a tubular part between which there is a dynamic interaction so that both parts show almost the same strain against a stress. However, there remains a need for tissue enginering scaffolds that are OQcapable of recapitulating the J-shaped curve behavior, and methods for making such scaffolds.

The present invention provides tissue engineering scaffolds that exhibit the same type of response to stress and strain, namely a J-shaped stress/strain curve, that is observed in native blood vessels, and methods of using and making the same.

### Summary of the Invention

The present invention concerns tissue engineering scaffolds and methods of making the same according to claims 1 and 2. In one aspect, the present invention provides methods of making a tissue engineering scaffold that includes two or more different tubular elements. The method includes the steps of (a) providing a first tubular element having an elastomeric element, an exterior surface, an interior luminal surface, and a first diameter; (b) dilating the first tubular element to a second diameter; (c) providing a second tubular element having a tensile element, an exterior surface and an interior luminal surface on the exterior surface of the dilated tubular element of step (b); (d) bonding the exterior surface of the dilated first tubular element of step (b) with the interior luminal surface of the second tubular element; and (e) decreasing the second diameter of the first tubular element to the first diameter of step (a) as defined in the claims.

In one embodiment, the first tubular element of step (a) and/or the second tubular element of step (c) is formed by electrospinning. In another embodiment, the first tubular element of step (a) is formed by electrospinning a material on a surface. In other embodiments, the second tubular element of step (c) is formed by electrospinning a material on the exterior surface of the dilated first tubular element, or by placing a pre-formed second tubular element on the exterior surface of the dilated first tubular element. In yet another embodiment, the first tubular element of step (a) is formed by electrospinning, and the second tubular element of step (c) is provided by placing a pre-formed second tubular element on the exterior surface of the dilated first tubular element.

The bonding step of (d) may comprise adhering the interior surface of the second tubular element to the exterior surface of the dilated first tubular element, or the bonding step (d) is performed after a second tubular element is electrospun on the exterior surface of the dilated first tubular element, or after the placement of a pre-formed second tubular element on the exterior surface of the dilated first tubular element, and includes the step of applying an additional layer of material on the outer surface of the second tubular element to allow adhesion sandwiching of the second tubular element between the first tubular element and the additional layer of material. The additional layer may be or may contain the same type of material that was used to form the first tubular layer. The outer layer or surface of the second tubular element of step (c) above is corrugated. In one embodiment, the corrugated second tubular element has a fibrous network in which the fiber direction is oriented circumferentially. In other embodiments, the outer layer or surface of a third, fourth, fifth, etc. tubular element is corrugated and/or has a fibrous network in which the fiber direction is oriented circumferentially.

In some embodiments, the providing step of (a) and/or the providing step of (c) includes electrospinning material on a mandrel. In another embodiment, the providing step of (c) comprises placing a pre-formed second tubular element over the dilated first tubular element of step (b). In one other embodiment, the providing step of (a) comprises electrospinning a material on a mandrel to form a first tubular elment, and the providing step of (c) comprises placing a pre-formed second tubular element over the dilated first tubular element of step (b).

The formation of additional tubular elements may include electrospinning on a mandrel, or placement of additional pre-formed tubular elements over the existing tubular element layers.

Steps (a) and (c) may include casting techniques. In one other example, step (a) involves the use of a cast corresponding to the first diameter and step (c) involves the use of a cast corresponding to the second diameter. In other examples, the formation of additional tubular elements includes casting, such as through the use of a cast corresponding to a diameter greater than or less than the second diameter of step (c); and/or through the use of a cast corresponding to a greater than or less than the diameter of the first diameter of step (a).

The methods may include the step of providing a continuum of tensile elements or continuum of stiffening within the second tubular element structure. In one example, the continuum is attributable to the varying morphology of the fibers within the second tubular element material.

The step of providing tubular elements contemplates the use of one or more of the following: casting, the use of pre-formed tubular elements, and electrospinning techniques.

The methods contemplate the provision of additional tubular elements over the first and second tubular elements, such as a third tubular element, a fourth tubular element, a fifth tubular element, etc. In all examples, each additional tubular element may include one or more elastomeric elements and/or one or more tensile elements. Those of skill in the art will appreciate the variety of techniques for providing additional tubular elements, including but not limited to those described herein.

In the present invention, the elastomeric element includes an elastomeric component having a first elastic modulus, and the tensile element includes a tensile component having a second elastic modulus that is greater than the first elastic modulus. In a preferred embodiment, the second elastic modulus is greater than the first elastic modulus by at least one order of magnitude.

In some embodiments, the elastomeric element includes a natural elastomeric component, a synthetic elastomeric component, or a natural elastomeric component and a synthetic elastomeric component. In one embodiment, the natural elastomeric component is elastin. In other embodiments, the natural elastomeric component is selected from the group consisting of elastin, resilin, abductin, and silk. In another embodiment, the synthetic elastomeric component may be selected from the group consisting of latex, a polyurethane (PU), polycaprolactone (PCL), poly-L-lactide acid (PLLA), polydiaxanone (PDO), poly(L-lactide-co-caprolactone) (PLCL), and poly(etherurethane urea) (PEUU).

In other embodiments, the tensile element includes a natural tensile component, a synthetic tensile component, or a natural tensile component and a synthetic tensile component. In one embodiment, the natural tensile component is collagen. In other embodiments, the natural tensile component is selected from the group consisting of collagen, cellulose, silk, and keratin. In another embodiment, the synthetic tensile component is selected from the group consisting of nylon, Dacron® (polyethylene terephthalate (PET)) Goretex® (polytetrafluoroethylene), polyester, polyglycolic acid (PGA), poly-lactic-co-glycolic acid (PLGA), and poly(etherurethane urea) (PEUU).

In another aspect, the present invention provides tissue engineering scaffolds made by the methods described herein having properties that mimic or are substantially similar to those of native blood vessels. In one embodiment, the present invention provides a tissue engineering scaffold having a mechanical response to stress and strain is substantially similar to that of a response by a native blood vessel that has (a) a first tubular element with an elastomeric element, an exterior surface and an interior luminal surface; and (b) a second tubular element with a tensile element, an exterior surface and an interior luminal surface in contact with the exterior surface of the first tubular element, wherein the tissue engineering scaffold's mechanical response to stress and strain is characterized by a J-shaped stress/strain curve as defined in the claims.

The scaffolds contemplate one or more additional tubular elements with the first and the second tubular elements. In some examples, the additional tubular element(s) are formed on the exterior surface of the second tubular element.

In another embodiment, the tissue engineering scaffold having a mechanical response to stress and strain substantially similar to that of a response by a native blood vessel has (a) a first tubular element with an elastomeric element, an exterior surface and an interior luminal surface; and (b) a second tubular element with a tensile element, an exterior surface and an interior luminal surface in contact with the exterior surface of the first tubular element, wherein the tissue engineering scaffold has (i) a circumferential tube elastic modulus 1 of about 0.1 MPa to about 0.5 MPa, (ii) a circumferential tube elastic modulus 2 of about 3.0 MPa to about 6.0 MPa; and (iii) a circumferential modulus transition of about 0.57 to about 1.12 as defined in the claims.

In other embodiments, the tissue engineering scaffold's mechanical response to stress and strain is characterized by a J-shaped stress/strain curve.

In some embodiments, the tissue engineering scaffold's mechanical response to stress and strain is attributable to synergy between the elastomeric element of the first tubular element and the tensile element of the second tubular element. In yet another embodiment, the elastomeric element confers elasticity to the tissue engineering scaffold and the tensile element confers rigidity to the tissue engineering scaffold synergistically. The second tubular element of the tissue engineering scaffold is corrugated. In one embodiment, the corrugated second tubular element has a fibrous network in which the fiber direction is oriented circumferentially. In one other embodiment, the axis of the corrugations is configured parallel to the axial direction of the scaffold. In some examples, the scaffolds contemplate one or more additional tubular elements, such as third, fourth, fifth, etc. tubular elements, where the outer layer or surface of a third, fourth, fifth, etc. tubular element is corrugated and/or has a fibrous network in which the fiber direction is oriented circumferentially. The present invention provides tissue engineering scaffolds where the elastomeric element contains an elastomeric component with a first elastic modulus and the tensile element contains a tensile component with a second elastic modulus that is greater than the first elastic modulus. In a preferred embodiment, the second elastic modulus is greater than the first elastic modulus by at least one order of magnitude.

In yet another embodiment, the present invention provides tissue engineering scaffolds where the elastomeric element has a natural elastomeric component, a synthetic elastomeric component, or a natural elastomeric component and a synthetic elastomeric component. In one embodiment, the natural elastomeric component is elastin. In other embodiments, the natural elastomeric component is selected from the group consisting of elastin, resilin, abductin, and silk. In other embodiments, the synthetic elastomeric component is selected from the group consisting of latex, a polyurethane (PU), polycaprolactone (PCL), poly-L-lactide acid (PLLA), polydiaxanone (PDO), poly(L-lactide-co-caprolactone) (PLCL), and poly(etherurethane urea) (PEUU). In some embodiments, the scaffolds of the present invention include (i) two or more different types of natural elastomeric components; and/or (ii) two or more different types of synthetic elastomeric components.

In other embodiments, the present invention provides tissue engineering scaffolds where the tensile element has a natural tensile component, a synthetic tensile component, or a natural tensile component and a synthetic tensile component. In one embodiment, the natural tensile component is collagen. In other embodiments, the natural tensile component is selected from the group consisting of collagen, cellulose, silk, and keratin. In another embodiment, the synthetic tensile component is selected from the group consisting of nylon, Dacron® (polyethylene terephthalate (PET)) Goretex® (polytetrafluoroethylene), polyester, polyglycolic acid (PGA), poly-lactic-co-glycolic acid (PLGA), and poly(etherurethane urea) (PEUU). In some embodiments, the tensile element of a scaffold includes (i) two or more different types of natural tensile components; and/or (ii) two or more different types of synthetic tensile components.

In another embodiment, a tissue engineering scaffold of the present invention has at least one of the following: (i) a pore gradient where the pore diameter gradually decreases from about 100 microns at the exterior surface of the second tubular element to about 5 to about 15 microns at the interior surface of the first tubular element; (ii) a circumferential tube toughness of about 0.45 MJ/m³ to about 1.0 MJ/m³; (iii) an axial tube toughness of about 0.1 MJ/m³ to about 0.5 MJ/m³; (iv) a tangent delta of about 0.05 to about 0.3; and (v) a storage modulus of about 400 MPa to about 0.12 MPa. In one embodiment, the pore gradient contributes to the enhancement of cell seeding capacity for a TE scaffold. In another embodiment, the axial toughness and/or circumferential toughness contribute to the rendering of a scaffold resistant to fracture or tearing. In one other embodiment, the viscoelasticity of a TE scaffold is characterized by the tangent delta and/or storage modulus values.

The TE scaffolds may include tubular elements in addition to a first and second tubular elements. Those of skill in the art will appreciate the variety of components that may be contained in the additional tubular elements, including but not limited to those described herein.

In additional embodiments, the disclosure provides methods of making tissue engineered scaffolds. In one embodiment, the method comprises the steps of (a) providing a first tubular element comprising an elastomeric element, an exterior surface, an interior luminal surface, and a first diameter; (b) dilating the first tubular element to a second diameter; (c) providing a second tubular element comprising a tensile element, an exterior surface and an interior luminal surface on the exterior surface of the first tubular element of step (b); (d) completing providing step (a) prior to completing providing step (c); (e) bonding the dilated tubular element of step (b) and the second tubular element of step (c); and (e) decreasing the second diameter of the first tubular element to the first diameter of step (a). In another embodiment, the tissue engineering scaffold comprises a zonal gradation at the interface between the first tubular element and the second tubular element. In another embodiment, the zonal gradation comprises a transitional zone of heterogeneity comprising the elastomeric element of the first tubular element and the tensile element of the second tubular element.

In one other embodiment of the disclosure, the method of making tissue engineered scaffolds comprises the steps of: (a) providing a first tubular element comprising an elastomeric element, an exterior surface, an interior luminal surface, and a first diameter; (b) dilating the first tubular element to a second diameter at a continuous rate; (c) providing a second tubular element comprising a tensile element, an exterior surface and an interior luminal surface on the exterior surface of the first tubular element of step (b) during dilating step (b); (e) bonding the dilated tubular element of step (b) and the second tubular element of step (c); and (e) decreasing the second diameter of the first tubular element to the first diameter of step (a). In another embodiment, the second tubular element comprises a continuum of tensile elements or a continuum of stiffening. In one other embodiment, the continuum of tensile elements engages at different strain values. In another embodiment, the bonding step (d) comprises binding of fibers of the second tubular element to the first tubular element, thereby providing the continuum. In one embodiment, the fibers of the second tubular element are linked prior to providing step (c). In another embodiment, the fibers engage at varying intervals upon strain depending upon the degree of kinking. In one embodiment, the fibers without a lesser amount of kinking straighten and engage before the fibers with a greater amount of kinking. In another embodiment, the fiber engagement leads to a gradual rounding of a stress/strain curve, thereby providing mechanical properties similar to a native blood vessel.

In another embodiment of the disclosure, the method further comprises (f) providing a third tubular element comprising an exterior surface and an interior luminal surface on the exterior surface of the second tubular element. In another embodiment, the method further comprises (g) providing a fourth tubular element comprising an exterior surface and an interior luminal surface on the exterior surface of the third tubular element. In one other embodiment, the method further comprises (h) providing a fifth tubular element comprising an exterior surface and an interior luminal surface on the exterior surface of the fourth tubular element. In one embodiment, the method further comprises providing one or more additional tubular elements comprising an exterior surface and an interior luminal surface, such that the interior luminal surface of each additional tubular element is contacted with the outermost tubular element. In one embodiment, the additional tubular element(s) comprise an elastomeric element. In one embodiment, the additional tubular element(s) comprise a tensile element. In another embodiment, the bonding step (e) comprises providing an additional tubular element comprising an elastomeric element, an exterior surface, and an interior luminal surface on the exterior surface of the second tubular element. In one other embodiment, the

In other embodiments, the present invention provides tissue engineering scaffolds. In one embodiment, the tissue engineering scaffold has a mechanical response to stress and strain is substantially similar to that of a response by a native blood vessel, the scaffold comprising (a) a first tubular element comprising an elastomeric element, an exterior surface and an interior luminal surface; and (b) a second tubular element comprising a tensile element, an exterior surface and an interior luminal surface in contact with the exterior surface of the first tubular element, wherein the tissue engineering scaffold comprises at least one of (i) a circumferential tube elastic modulus 1 of about 0.1 MPa to about 0.5 MPa, (ii) a circumferential tube elastic modulus 2 of about 3.0 MPa to about 6.0 MPa; and (iii) a circumferential modulus transition of about 0.57 MPa to about 1.12 MPa; (iv) a pore gradient where the pore diameter gradually decreases from about 100 microns at the exterior surface of the second tubular element to about 5 to about 15 microns at the interior surface of the first tubular element; (v) a circumferential tube toughness of about 0.45 MJ/m³ to about 1.0 MJ/m³; (vi) an axial tube toughness of about 0.1 MJ/m³ to about 0.5 MJ/m³; (vii) a tangent delta of about 0.05 to about 0.3; and (viii) a storage modulus of about 400 MPa to about 0.12 MPa, or any combination thereof. In another embodiment, the the tissue engineering scaffold's mechanical response to stress and strain is characterized by a J-shaped stress/strain curve. In one embodiment, the tissue engineering scaffold is accessible to cells. In another embodiment, the tissue engineering scaffold is fracture-resistant. In yet another embodiment, the tissue engineering scaffold is viscoelastic. The present invention provides a tissue engineering scaffold comprising (a) a first tubular element comprising an elastomeric element, an exterior surface and an interior luminal surface; and (b) a corrugated second tubular element comprising a tensile element, an exterior surface and an interior luminal surface in contact with the exterior surface of the first tubular element.

In yet further embodiments, the present disclosure provides tissue engineered blood vessels (TEBVs). In one embodiment, the TEBV comprises (a) a first tubular element comprising (i) an elastomeric element, (ii) an exterior surface, (iii) an interior luminal surface; (b) a second tubular element comprising (i) a tensile element, (ii) an exterior surface, (iii) an interior luminal surface in contact with the exterior surface of the first tubular element, and (c) a first cell population, wherein the TEBV's mechanical response to stress and strain is characterized by a J-shaped stress/strain curve. In another embodiment, the TEBV comprises (a) a first tubular element comprising (i) an elastomeric element, (ii) an exterior surface, (iii) an interior luminal surface; (b) a second tubular element comprising (i) a tensile element, (ii) an exterior surface, (iii) an interior luminal surface in contact with the exterior surface of the first tubular element, and (c) a first cell population, wherein the TEBV comprises at least one of (i) a circumferential tube elastic modulus 1 of about 0.1 MPa to about 0.5 MPa, (ii) a circumferential tube elastic modulus 2 of about 3.0 MPa to about 6.0 MPa; and (iii) a circumferential modulus transition of about 0.57 MPa to about 1.12 MPa; (iv) a pore gradient where the pore diameter gradually decreases from about 100 microns at the exterior surface of the second tubular element to about 5 to about 15 microns at the interior surface of the first tubular element; (v) a circumferential tube toughness of about 0.45 MJ/m³ to about 1.0 MJ/m³; (vi) an axial tube toughness of about 0.1 MJ/m³ to about 0.5 MJ/m³; (vii) a tangent delta of about 0.05 to about 0.3; and (viii) a storage modulus of about 400 MPa to about 0.12 MPa. In another embodiment, the TEBV is characterized by a J-shaped stress/strain curve. In one embodiment, the TEBV's mechanical response to stress and strain is attributable to synergy between the elastomeric element of the first tubular element and the tensile element of the second tubular element. In another embodiment, the elastomeric element confers elasticity to the TEBV and the tensile element confers rigidity to the TEBV synergistically. The second tubular element is corrugated. In another embodiment, the corrugated second tubular layer comprises a fibrous network in which the fiber direction is oriented circumferentially. In another embodiment, the elastomeric element comprises an elastomeric component with a first elastic modulus and the tensile element comprises a tensile component with a second elastic modulus that is greater than the first elastic modulus. In other embodiments, the second elastic modulus is greater than the first elastic modulus by at least one order of magnitude. In another embodiment, the elastomeric element comprises a natural elastomeric component. In other embodiments, the elastomeric element comprises a synthetic elastomeric component. In another embodiment, the elastomeric element comprises a natural elastomeric component and a synthetic elastomeric component. In one embodiment, the natural elastomeric component is selected from the group consisting of elastin, resilin, abductin, and silk. In another embodiment, the synthetic elastomeric component is selected from the group consisting of latex, a polyurethane (PU), polycaprolactone (PCL), poly-L-lactide acid (PLLA), polydiaxanone (PDO), poly(L-lactide-co-caprolactone) (PLCL), and poly(etherurethane urea) (PEUU). In one embodiment, the tensile element comprises a natural tensile component. In one embodiment, the tensile element comprises a synthetic tensile component. In one embodiment, the tensile element comprises a natural tensile component and a synthetic tensile component. In one embodiment, the natural tensile component is selected from the group consisting of collagen, cellulose, silk, and keratin. In one embodiment, the synthetic tensile component is selected from the group consisting of nylon, Dacron® (polyethylene terephthalate (PET)) Goretex® (polytetrafluoroethylene), polyester, polyglycolic acid (PGA), poly-lactic-co-glycolic acid (PLGA), and poly(etherurethane urea) (PEUU). In one embodiment of the disclosure, the first cell population is within the second tubular element and/or on the exterior surface of the second tubular element. In one embodiment, the first cell population is a smooth muscle population. In one embodiment, the tubular scaffold further comprises a second cell population. In another embodiment, the second cell population is on and/or within the interior luminal surface of the first tubular element. In one embodiment, the second cell population is an endothelial cell population. The present disclosure provides a TEBV comprising (a) a first tubular element comprising (i) an elastomeric element, (ii) an exterior surface, (iii) an interior luminal surface; (b) a corrugated second tubular element comprising (i) a tensile element, (ii) an exterior surface, (iii) an interior luminal surface in contact with the exterior surface of the first tubular element, and (c) a first cell population.

In yet further embodiments, the present disclosure provides a method of making tissue engineered blood vessels (TEBVs) comprising the steps of: (a) providing a first tubular element comprising an elastomeric element, an exterior surface, an interior luminal surface, and a first diameter; (b) dilating the first tubular element to a second diameter;(c) providing a second tubular element comprising a tensile element, an exterior surface, a first cell population on the exterior surface of and/or within the second tubular element and an interior luminal surface on the exterior surface of the first tubular element of step (b); (d) bonding the dilated tubular element of step (b) and the second tubular element of step (c); (e) decreasing the second diameter of the first tubular element to the first diameter of step (a) to provide the TEBV; (f) culturing the TEBV. In one embodiment, the second tubular element of step (c) is corrugated. In one embodiment, the corrugated second tubular element comprises a fibrous network in which the fiber direction is oriented circumferentially. In one embodiment, the providing step of (a) comprises electrospinning an elastomeric component on a mandrel and the providing step of (c) comprises (i) electrospinning a tensile component on a mandrel, and (ii) electrospraying the first cell population on a mandrel. In one embodiment, the electrospinning step of (i) and electrospraying step of (ii) are concurrently performed. In one embodiment, the method further comprises step (f) seeding the interior luminal surface of step (a) with a second cell population. In one embodiment, the second cell population is an endothelial cell population. In one embodiment of the disclosure, the elastomeric element comprises an elastomeric component with a first elastic modulus and the tensile element comprises a tensile component with a second elastic modulus that is greater than the first elastic modulus. In one embodiment, the second elastic modulus is greater than the first elastic modulus by at least one order of magnitude. In one embodiment, the elastomeric element comprises a natural elastomeric component. In one embodiment, the elastomeric element comprises a synthetic elastomeric component. In one embodiment, the elastomeric element comprises a natural elastomeric component and a synthetic elastomeric component. In one embodiment, the natural elastomeric component is elastin. In one embodiment, the synthetic elastomeric component is selected from the group consisting of polycaprolactone (PCL), poly-L-lactide acid (PLLA), polydiaxanone (PDO), poly(L-lactide-co-caprolactone) (PLCL), and poly(etherurethane urea) (PEUU). In one embodiment, the tensile element comprises a natural tensile component. In one embodiment, the tensile element comprises a synthetic tensile component. In one embodiment, the tensile element comprises a natural tensile component and a synthetic tensile component. In one embodiment, the natural tensile component is collagen. In one embodiment, the synthetic tensile component is selected from the group consisting of polyglycolic acid (PGA), poly-lactic-co-glycolic acid (PLGA), and poly(etherurethane urea) (PEUU). In one embodiment, the method further comprises contacting the TEBV of step (e) with at least one additional cell population prior to step (f) or after step (f). In one embodiment, the culturing step (f) comprises conditioning by pulsatile and/or steady flow in a bioreactor.

In yet further embodiments, the disclosure is directed to tissue engineering scaffolds (TE scaffolds) or tissue engineered blood vessels (TEBVs) made by the methods disclosed herein, or any other suitable method, where the TE scaffolds or TEBVs have a zonal gradation at the interface between the first tubular element and the second tubular element. In other embodiments, the zonal gradation comprises a transitional zone of heterogeneity that includes material from the elastomeric element of the first tubular element and material from the tensile element of the second tubular element.

In certain embodiments, the disclosure is directed to tissue engineering scaffolds (TE scaffolds) or tissue engineered blood vessels (TEBVs) made by the methods disclosed herein, or any other suitable method, where the second tubular element of the TE scaffolds or TEBVs have a continuum of tensile elements or a continuum of stiffening. In other embodiments, the continuum of tensile elements engages at different strain values. In one embodiment, the continuum is attributable to the varying morphologies of the individual fibers of the second tubular element material.

In some embodiments, the tissue engineering scaffolds (TE scaffolds) or tissue engineered blood vessels (TEBVs) have a zonal gradation at the interface between the first tubular element and the second tubular element and the second tubular element has a continuum of tensile elements. In other embodiments, the zonal gradation comprises a transitional zone of heterogeneity that includes material from the elastomeric element of the first tubular element and material from the tensile element of the second tubular element and/or the continuum of tensile elements engages at different strain values.

### Brief Description of the Drawings

Figure 1 shows the stress/strain relationship of a native blood vessel, a native blood vessel minus collagen (labeled "Elastin"), and a native blood vessel minus elastin (labeled "Collagen").
Figure 2 shows the "J" shaped curve approximated by distinguishing two linear regions relating to two different moduli.
Figure 3A-B illustrates the creation of tubular structures from electrospinning and casting.
Figure 4 illustrates the creation of tubular architectures by electrospinning. Figures 4A-B illustrate an electrospinning technique for providing a tissue engineered scaffold. Figure 4C illustrates a sudden transition between lamina (top) and a transitional mixing of layers (bottom). Figure 4D illustrates an electrospinning technique for achieving zonal gradation in a tissue engineering scaffold. Fig. 4E depicts an alternative embodiment of the expanding mandrel process.
Figure 5A-B illustrates an expanding mandrel capable of continuous diameter change during rotation.
Figure 6 illustrates the application of a thin tensile mesh over an expanded elastic lamina.
Figure 7 illustrates fiber morphologies from felt materials.
Figure 8 shows the stress/strain relationship of a latex/PDO architecture.
Figure 9 shows the stress/strain relationship of a latex/Vicryl architecture.
Figure 10 shows the stress/strain relationship of PDO and Vicryl.
Figure 11 shows the stress/strain relationship of latex.
Figure 12 shows the stress/strain relationship of tubes containing PGA and/or PU.
Figure 13 shows the stress strain relationships of a tube containing PU and PGA, and native porcine carotid arteries.
Figure 14A-B shows a representative tubular scaffold of sutured material around a latex tube.
Figure 15A-B shows a representative corrugated scaffold.
Figure 16A-B shows cross-sections of representative corrugated scaffolds.
Figure 17 shows the stress/strain relationship of tubes containing PLCL/PGA and PU/PGA.
Figure 18 shows the pressure/volume relationship of tubes containing PLCL/PGA and PU/PGA.
Figure 19A-C depicts the concept of tunability for tubular scaffolds. A - Failure of the tensile element and failure of the elastic element coincides; B - Failure of the elastic element prior to failure of the tensile element; C - Hypothetical failure of the tensile element prior to failure of the elastic element.
Figure 20 shows the histochemistry of tubular scaffolds.
Figure 21A-E shows cell staining of segments of the tubular scaffolds following cell seeding and bioreactor conditioning.
Figure 22 shows the results of a whole blood clotting assay of the cell-seeded, bioreactor-conditioned tubular scaffolds.
Figure 23 shows the schematic of a bioreactor used to condition tubular scaffolds.

### DETAILED DESCRIPTION OF THE PREFERRED EMBODIMENTS

The present invention concerns tissue engineering (TE) scaffolds and methods of making the same. In particular, the invention provides TE scaffolds having properties that are substantially similar to those of native blood vessels. For example, the TE scaffolds of the present invention exhibit a mechanical response to stress and strain, namely a J-shaped stress/strain curve, that is substantially similar to that of a native blood vessel.

### 1. Definitions

Unless defined otherwise, technical and scientific terms used herein have the same meaning as commonly understood by one of ordinary skill in the art to which this invention belongs.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

Other relevant information is available from text books in the field of tissue engineering, such as, for example, Palsson, Bernhard O., Tissue Engineering, Prentice Hall, 2004 and Principles of Tissue Engineering, 3rd Ed. (Edited by R Lanza, R Langer, & J Vacanti), 2007.

The term "tissue engineering scaffold" or "TE scaffold" as used herein refers to a tubular structure that is laminated or multi-layered and characterized by an ability to respond to stress and strain in a manner that is substantially similar to a native blood vessel. For example, the scaffold's mechanical response to stress and strain is preferably characterized by a J-shaped stress/strain curve. The properties of the scaffolds of the present invention make them suitable for use as a framework for a blood vessel scaffold.

The term "tissue engineered blood vessel" or "TEBV" or "blood vessel scaffold" as used herein refers to a tissue engineering scaffold as defined above and described herein that has been further manipulated to render it suitable for transplantation into a mammalian subject in need. For example, the TEBV may be formed by manipulating a tissue engineering scaffold to add one or more cell populations by the methods described herein, or by any other suitable method. Those of ordinary skill in the art will appreciate that the present invention pertains to many types of blood vessels, including without limitation, the carotid artery, the subclavian artery, the celiac trunk, the mesenteric artery, the renal artery, the iliac artery, arterioles, capillaries, venules, the subclavian vein, the jugular vein, the renal vein, the iliac vein, the venae cavae. In addition, a TEBV of the present invention may also be arteriovenous shunt (AV shunt) or an inter-positional blood vessel graft.

The term "elastomeric element" refers to a material characterized by it ability to respond to stress with large-scale deformations that are fully recoverable and repeatable. The elastomeric element may comprise a natural component, a synthetic component, or a mixture of natural and synthetic components.

The term "tensile element" refers to a material that is characterized by very little ability to elongate when stressed. The tensile element may comprise a natural component, a synthetic component, or a mixture of natural and synthetic components.

The term "synthetic component" as used herein refers to a component that does not normally exist in nature. Generally, synthetic components are not normally present in a native blood vessel, but nonetheless have the potential to exhibit native vessel-like properties with respect to mechanics and cellular behavior. A synthetic component may be part of a tissue engineering scaffold and/or a TEBV, as described herein, that may optionally include a natural component (as defined below). Synthetic components may be elastomeric or tensile in nature.

The term "natural component" as used herein refers to a substance that exists in nature or is derived from a substance that exists in nature, regardless of its mode of preparation. Thus, for example, a "natural component" may be a native polypeptide isolated and purified from its native source, or produced by recombinant and/or synthetic means. Natural components may be present in a native blood vessel and therefore have the potential to exhibit native vessel-like properties with respect to mechanical and cellular behavior. In certain embodiments, natural components may be elastomeric or tensile in nature.

The term "corrugated" as used herein, refers to a structure containing a tensile component characterized by corrugations, undulations, and/or kinks on one or more of its surfaces. This structure is generally in the form of a thin layer or lamina made up of a fibrous network in which the fiber direction is generally oriented circumferentially. In addition, the axis of the corrugations is configured to be parallel to the axial direction of the structure, e.g., a tubular tissue engineering scaffold.

The term "mechanical response" or "biomechanical response" as used herein refers to the behavior exhibited by a native blood vessel, blood vessel scaffold, or tissue engineering scaffold when subjected to stress and strain. The behavior upon exposure to stress and strain is preferably characterized by one or more of the following: (i) a J-shaped stress/strain curve; (ii) viscoelasticity; and (iii) resistance to tearing or fracturing.

The term "substantially similar to a native blood vessel" as used herein refers to a scaffold having mechanical properties that closely mimic or resemble those of a native blood vessel. Those of ordinary skill in the art will appreciate that several parameters can be characterized and measured to demonstrate this substantial similarity. Important parameters for providing the tissue engineering scaffolds of the present invention with mechanical behavior that is substantially similar to a native blood vessel, including a J-shaped stress/strain curve, are the scaffold's circumferential tube elastic modulus 1, circumferential tube elastic modulus 2, and the circumferential tube's modulus transition. In a preferred embodiment, other parameters also contribute to the desired mechanical behavior or response of the scaffolds to stress and strain and/or their capacity to serve as a vascular graft, including, without limitation, compliance, Young's or elastic modulus, burst pressure, wall thickness, porosity, pore diameter, pore gradient, fiber diameter, breaking strain (axial and/or circumferential), breaking stress (axial and/or circumferential), toughness (axial and/or circumferential), axial tube elastic moduli 1 and 2, the axial tube's elastic modulus transition, and viscoelastic properties such as those demonstrated by particular tangent delta (tan delta) and storage modulus values.

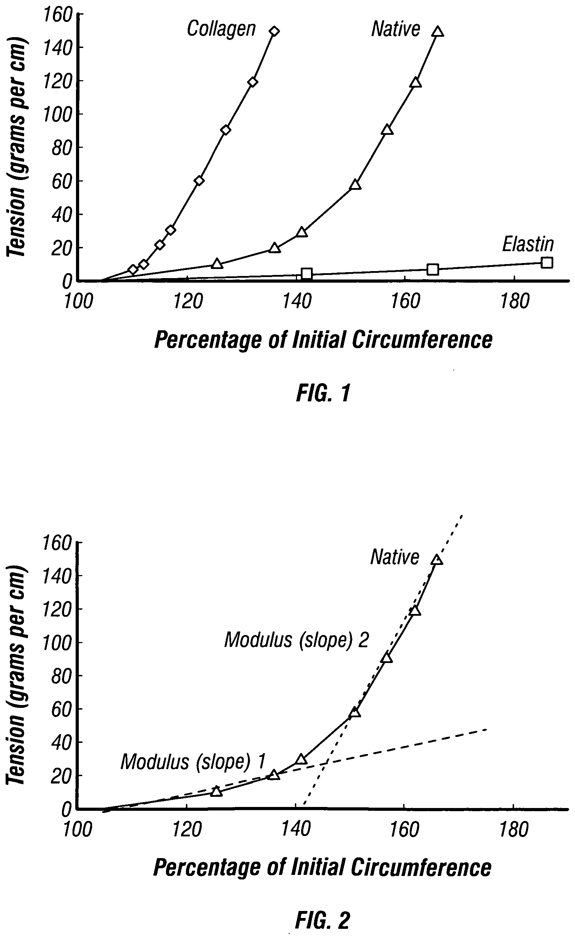

The term "J-shaped curve" as used herein refers to the shape of the curve where stress (force per unit area of material or pressure) is plotted on the y-axis and strain (change in length over the original length or displacement) is plotted on the x-axis. The J-shaped curve is a mechanical response to stress and strain that is inherent to native arteries arising from the synergistic interplay of collagen and elastin, as depicted in Figure 1.

The term "compliance" as used herein is defined by the formula C=Δ(delta)V/Δ(delta)P (the slope) on a pressure (x-axis)/volume (y-axis) curve. It is the measure of "softness" in a material and is the inverse of "stiffness". Typically, C is mL/mm Hg where V is volume (mL) and P is pressure (mm Hg).

The term "Young's modulus" or "Elastic modulus" as used herein is defined as a parameter for stiffness. It is derived from the slope of a stress (y-axis)/strain (x-axis) curve. In the case of a non-linear "J" shaped curve, the elastic modulus can be modeled as two separate intersecting slopes, in which the first slope is derived from the initial quasi-linear segment (elastic modulus 1) and the second slope is derived from the later quasi-linear segment (elastic modulus 2). Figure 2 illustrates this concept.

The term "elastic modulus 1 to elastic modulus 2 transition" or "modulus 1 to modulus 2 transition" or "elastic modulus transition" as used herein refers to the range over which the slope of elastic modulus 1 transitions or changes to the slope of elastic modulus 2. The unit of expression for this parameter is a strain value at which the slope occurs. This is illustrated in Fig. 2 where the straight lines represented by Modulus (slope) 1 and Modulus (slope) 2 intersect. In the curve showing the response in native blood vessels, the transition is illustrated by the segment of the curve indicating a change from the Modulus (slope) 1 to the Modulus (slope) 2.

The term "compliance mismatch" as used herein refers to the union of two materials with differing measures of softness/stiffness (i.e. compliance/Young's modulus or Elastic modulus).

The term "porosity" as used herein is defined as the ratio of pore volume in a scaffold to the total volume of the scaffold, and may be expressed as a percentage porosity. Alternatively, porosity may be the percentage ratio of pore area in a scaffold to the total area of the scaffold.

The term "burst pressure" as used herein is defined as the difference in pressure between the interior and exterior of a tubular scaffold which the scaffold can withstand before at least a partial disintegration of the scaffold occurs.

The term "wall thickness" as used herein is defined as the depth or extent from the exterior surface of a tubular scaffold to its interior luminal surface.

The term "pore diameter" as used herein is defined as the average diameter of the pores within a scaffold of the present invention.

The term "pore gradient" as used herein is defined as a linear change in pore diameter size from one surface to another. The pore diameter size will gradually decrease within a layer of a tubular element. For instance, the size can decrease from one surface, such as the adventitial or exterior surface of a tubular element, to another surface, such as a luminal or interior surface of the tubular element.

The term "fiber diameter" as used herein is defined as the average diameter of the fibers of a scaffold of the present invention.

The term "breaking strain" as used herein is defined as strain at fracture in a material.

The term "breaking stress" as used herein is defined as stress at failure in a material.

The term "toughness" as used herein is defined as the energy required to fracture a material, the calculated area under a stress/strain curve to failure.

The term "tangent delta" as used herein is defined as an indicator of the relative amounts of energy stored and lost in a tubular scaffold and is typically used to characterize molecular relaxations and identify rheological transformations.

The term "storage modulus" as used herein is defined as the ability of a material to store mechanical energy, and is typically used to characterize molecular relaxations.

The term "kink radius" as used herein is defined as the radius at which a kink forms in a flexed tubular structure.

The term "zonal gradation" as used herein is defined as a gradual gradient in a laminate structure having at least two different layers; where each layer contains a different type of material; and where the gradient exists between the layers and is a zone of heterogeneity as between different materials. For example, the zone of heterogeneity may contain material from an elastomeric element and material from a tensile element.

The term "smooth muscle cell" as used herein refers to a cell that makes up non-striated muscle that is found in the walls of hollow organs (e.g. bladder, abdominal cavity, uterus, gastrointestinal tract, vasculature, etc.) and is characterized by the ability to contract and relax. Vascular smooth muscle cells are found throughout the tunica media (thickest layer of a blood vessel), which contains a circularly arranged elastic fiber and connective tissue. As described below, smooth muscle cell populations can be isolated from a variety of sources.

The term "endothelial cell" as used herein refers to a cell that is suitable for seeding on the a scaffold of the present invention, either on the interior luminal surface or within the scaffold. Endothelial cells cover the interior or luminal surface of native blood vessels and serve multiple functions including, but not limited to, the prevention of thrombosis and the prevention of tissue in-growth and unwanted extracellular matrix production. As described below, endothelial cell populations for seeding onto scaffolds of the present invention can be isolated from a variety of sources including, without limitation, the vascular parenchyma, circulating endothelial cells and endothelial cell precursors such as bone marrow progenitor cells, peripheral blood stem cells and embryonic stem cells.

The term "cell population" as used herein refers to a number of cells obtained by isolation directly from a suitable tissue source, usually from a mammal, and subsequent culturing *in vitro.* Those of ordinary skill in the art will appreciate that various methods for isolating and culturing cell populations for use with the present invention and the various numbers of cells in a cell population that are suitable for use in the present invention.

The term "mammal" as used herein refers to any animal classified as a mammal, including, without limitation, humans, non-human primates, domestic and farm animals, and zoo, sports or pet animals such horses, pigs, cattle, dogs, cats and ferrets, *etc.* In a preferred embodiment of the invention, the mammal is a human.

The term "non-human animal" as used herein includes, but is not limited to, mammals such as, for example, non-human primates, rodents (*e.g.*, mice and rats), and non-rodent animals, such as, for example, rabbits, pigs, sheep, goats, cows, pigs, horses and donkeys. It also includes birds (*e.g.,* chickens, turkeys, ducks, geese and the like). The term "non-primate animal" as used herein refers to mammals other than primates, including but not limited to the mammals specifically listed above.

A "cardiovascular disease" or "cardiovascular disorder" is used herein in a broad, general sense to refer to disorders or conditions in mammals characterized by an abnormality in the function of the heart or blood vessels (arteries and veins) and affecting the cardiovascular system, particularly those diseases related to atherosclerosis. Such diseases or disorders are particularly amenable to treatment using a TEBV described herein as a bypass vascular graft. Such grafts include, without limitation, coronary artery bypass graft (CABGs), peripheral bypass grafts, or arteriovenous shunts. Examples of cardiovascular disorders include, without limitation, those conditions caused by myocardial ischemia, a heart attack, a stroke, a transmural or non-transmural myocardial infarction, an acute myocardial infarction, peripheral vascular disease, coronary artery disease, coronary heart disease, an arrhythmia, sudden cardiac death, a cerebrovascular accident such as stroke, congestive heart failure, a life-threatening dysrhythmia, cardiomyopathy, a transient ischemic attack, an acute ischemic syndrome, or angina pectoralis, acute coronary stent failure, or a combination thereof. Other examples of such disorders include,without limitation, thrombotic conditions such as pulmonary embolism, acute thrombosis of the coronary arteries, myocardial infarction, acute thrombosis of the cerebral arteries (stroke) or other organs.

### 2. J-shaped curve stress/strain response

Figure 1 depicts a J-shaped curve, which is a mechanical response to stress and strain inherent to native arteries that arises from the synergistic interplay of two major structural proteins, collagen and elastin (Roach et al. (1957) Can. J. Biochem. Physiol. 35:681-690). Native vessel mechanics are nonlinear and chacterized by a "J" shaped curve on a force (stress)/displacement (strain) diagram resulting from the synergistic interplay of collagen and elastin (Figure 2). The presence of both collagen and elastin in arteries gives them their profound nonlinear behavior. If a native artery has its elastin extracted leaving collagen as the remaining primary structural protein, the mechanical response becomes much stiffer. Conversely, if a native artery is treated to remove collagen, the predominant structural protein is elastin, and the mechanics reflect a linear elastic character. The "J" shaped curve of the native artery is non-linear behavior resulting from the combined effects of both collagen and elastin, the major structural proteins present in arteries (Gosline & Shadwick (1998) American Scientist. 86:535-541).

In this biological composite, collagen behaves as a high stiffness, low elasticity component while elastin behaves as the high elasticity, low stiffness element. Collagen is a tensile element with very little ability to elongate when stressed and thus is particularly suited to roles in tissues such as tendon and ligament. Elastin, however, is characterized by its ability to respond to stress with large-scale deformations that are fully recoverable and repeatable. These characteristics of elastin make it suitable for tissues that require some sort of recoil or restoring force such as skin, arteries, and lungs.

One important failure mode associated with the loss of patency in vascular grafts is intimal hyperplasia (IH), which is characterized by tissue in-growth at the suture line. IH is known to be caused by the compliance mismatch of the resulting interface between two vascular segments of very different mechanical properties (O'Donnell et al. (1984) J. Vasc. Surg. 1:136-148; Sayers et al. (1998) Br. J. Surg. 85:934-938; Stephen et al. (1977) Surgery. 81:314-318; Teebken et al. (2002) Eur. J. Vasc. Endovasc. Surg. 23(6):475-85; Kannan et al. (2005) J. Biomed. Mater. Res Part B - Appl Biomater 74B(1):570-81; Walpoth et al. (2005) Expert Rev. Med. Dev. 2(6):647-51)). This interface zone develops unnatural hydrodynamic conditions that set the stage for pathological processes and eventual occlusion (loss of patency) of the graft.

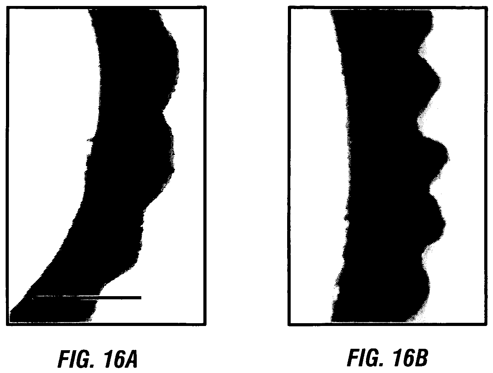

Although compliance matching has been recognized as important, given the nonlinear behavior of native arteries, it is unlikely that a significant match can occur with the specification of only one slope (portion of the mechanical response curve). The general trend for determining compliance (and stiffness) appears to be through consideration of only the initial quasi-linear segment from the respective graphs (Sanders et al. U.S. Published Patent Application 2003/0211130 (Figure 16); Lee et al. (2007) J Biomed Mater Res A. [Epub ahead of print PMID: 17584890]; Smith et al. (2007) Acta Biomater. [Epub ahead of print, PMID: 17897890]). However, by ignoring what happens after this initial quasi-linear segment, important information is lost. The "J" shaped curve is nonlinear as shown in Figure 1 and therefore could be modeled as two separate slopes intersecting. Figure 2 illustrates this concept showing one "J"-shaped curve approximately by distinguishing two linear regions which relate to two different moduli (stiffnesses). The same approach can be used on pressure/volume graph for compliance. Therefore, where compliance is concerned, the present invention considers measurements taken not only during the initial quasi-linear segment on the stress/strain graph, but also measurements taken after this initial segment.

The "J"-shape of the curve does not merely represent the chance mechanical behavior resulting from the particular choice of materials employed in the construction of native vessels. Rather, the shape itself denotes a particular resistance to the formation of aneurysms (Shadwick (1998) American Scientist. 86:535-541). Additionally, mimicking native vessel mechanical behavior provides macroscopic benefits, namely modulation of compliance mismatch. Others have shown that many different types of cells are sensitive to the microscopic mechanical environment in which they are seeded. This includes the mechanical properties of the substrate the cells are seeded on as well as the stress imparted to cells via factors affecting tissues such as compression (e.g. cartilage in a knee joint), cyclical strain (e.g. a blood vessel experiencing pulsatile flow), etc. (Georges et al. (2006) Biophys. J. 90(8):3012-18; Engler et al. (2004) J. Cell Biol. 13;166(6):877-87; Rehfeldt et al. (2007) Adv. Drug. Deliv. Rev. Nov 10;59(13):1329-39; Peyton et al. (2007) Cell Biochem. Biophys. 47(2):300-20). For example, vascular smooth muscle cells are sensitive to certain strain regimes in vascular tissue (Richard et al. (2007) J. Biol. Chem. 282(32):23081-8). In addition, cells in tendons, bone, and virtually every tissue in the body are exquisitely tuned to the microscopic mechanical environment which they inhabit, which provides yet another compelling reason to closely mimic the behavior of native tissue. Departures from the expected mechanical properties can send cells down different developmental pathways, or ultimately lethal pathways involving necrosis or apoptosis.

### 3. Tissue engineering (TE) scaffolds

Native blood vessels have a multi-layered or laminated structure. For example, an artery has three layers: an innermost layer called the intima that comprises macrovascular endothelial cells lining the luminal surface, a middle layer called the media that comprises multiple sheets of smooth muscle cells, and the outer layer called the adventia that contains loose connective tissue, smaller blood vessels, and nerves. The intima and media are separated by a basement membrane.

Specialized architectural features (undulations, corrugations, kinks) in native vessels facilitate parallel arrangements of collagen and elastin lamina being mechanically engaged to differing degrees at differing strains. Native arteries possess elastic laminae that are concentrically arranged in a circumferential direction. Such laminae are corrugated. In theory, the corrugations of elastic laminae could entrain surrounding collagen layers and impart similar geometry to them but this is not typically observed. Moreover, histology shows that elastic laminae are typically surrounded by concentrations of glycosaminoglycans (GAGs). For example, a 2007 report by Dahl et al. report the comparison of a tissue engineered blood vessel with a native artery, in which corrugated elastin laminae were clearly visualized in each through the use of representative Movat's stain and Verheoff-Van Gieson's stain (Annals of Biomedical Engineering 2007 Mar;35(3):348-55). Therefore, the typical observation in native arteries are corrugations in elastic laminae but no corrugations in surrounding collagen layers. An exception to this is an unusual architecture documented in fin whales, where a novel connective tissue design is present in which the collagenous component, which happens to be the tensile element, is highly corrugated (Gosline 1998 *supra*).

As described herein, the present invention involves tissue engineering scaffolds and methods of making the same that take a reverse approach to what is typically seen in native arteries, that is, the tensile layer of the scaffold has corrugations but not the elastic layer. This approach is advantageous because it is easier to impart corrugations within a tensile layer than it is to impart them in an elastic layer.

The tissue engineering scaffolds disclosed herein have a mutli-layered or laminated structure. In one embodiment, the scaffold includes (a) a first tubular element that contains an elastomeric element, an exterior surface and an interior luminal surface; and (b) a second tubular element that contains a tensile element, an exterior surface and an interior luminal surface in contact with the exterior surface of the first tubular element.

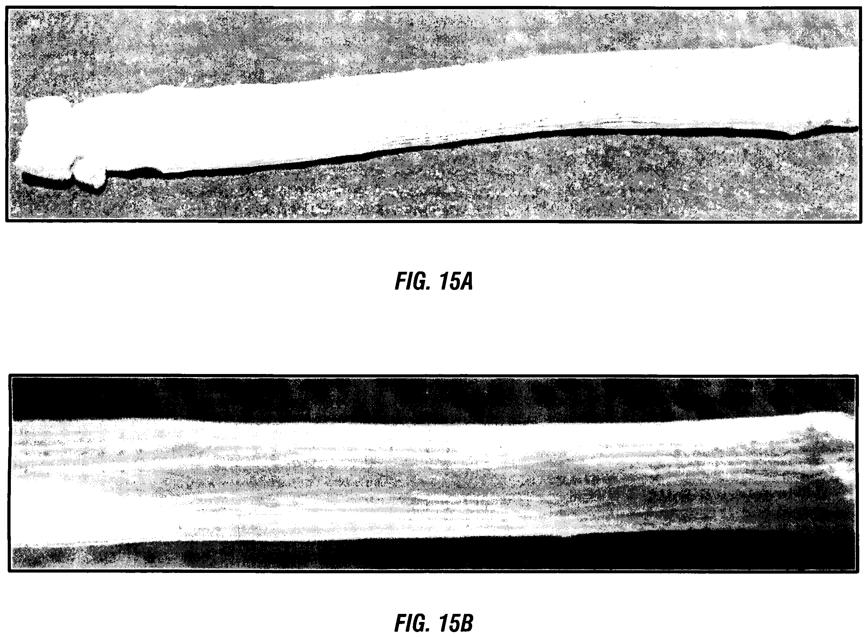

In another embodiment, the second tubular element is corrugated. The corrugations present in the tissue engineering scaffolds described herein are exemplified by Figure 15A-B showing their appearance on the outer surface of the scaffolds.

In other embodiments, the corrugated second tubular element has a fibrous network in which the fiber direction is oriented circumferentially. Figure 16A-B shows a cross-sectional view of the circumferentially uniform nature of the corrugations

Additional tubular elements may be added over the first and second tubular elements.

The interior luminal surface of the first tubular element and the exterior surface of the second tubular element are both accessible for further manipulation, such as, for example in the formation of a TEBV. As described below, the tissue engineering scaffolds of the present invention may be used to make tissue engineered blood vessels (TEBVs) by incorporating one or more cell populations into the scaffold. The laminated construction of the scaffolds provides a more natural vessel morphology which might facilitate the expected partitioning of cell populations, such as smooth muscle cells, endothelial cells, and fibroblasts.

The elastomeric element of the scaffolds described herein confers to the scaffold an ability to respond to stress with large-scale deformations that are fully recoverable and repeatable. The elastomeric elements have an elastomeric component that may be a natural component, a synthetic component, a mixture of more than one natural component, a mixture of more than one synthetic component, a mixture of natural and synthetic components, or any combination thereof. In general, an organic or natural component is a protein that is normally present in native tissue structures, or can be derived from native tissue structures, or can be produced recombinantly or synthetically based on the known nucleic acid sequence encoding the protein and/or its amino acid sequence. For example, elastin is naturally present in arteries and may be utilized as a natural component in the blood vessel scaffolds of the present invention. A natural component may be part of a TE scaffold and/or a TEBV, as described herein, that also includes or does not include a synthetic component.

In some embodiments, the elastomeric element of the first tubular element includes an organic or natural component, such as an elastic protein, including without limitation, elastin, gluten, gliadin, abductin, spider silks, and resilin or pro-resilin (Elvin et al. (2005) Nature. Oct 12:437(7061):999-1002). Those of ordinary skill in the art will appreciate other natural elastic proteins that may be suitable for use in the scaffolds of the present invention.

The use of natural materials provides an advantage when the intact blood vessel scaffold is subjected to further manipulation for the purpose of constructing a tissue engineered blood vessel. For example, when a particular cell population is cultured on or seeded on the scaffold, the natural elastin protein present in the scaffold encourages proper cell interaction with the scaffold.

In other embodiments, the elastomeric element includes a synthetic component. Examples of synthetic elastomeric components, include without limitation, latex, a polyurethane (PU), polycaprolactone (PCL), poly-L-lactide acid (PLLA), polydiaxanone (PDO), poly(L-lactide-co-caprolactone) (PLCL), and poly(etherurethane urea) (PEUU).

In one embodiment, the present disclosure contemplates first tubular elements in which the elastomeric element includes a natural elastic component and a synthetic elastic component.

The tensile element of the scaffolds described herein confers to the scaffold rigidity or tensility that allows the scaffold to resist elongation in response to stress. The tensile elements have a tensile component that may be a natural component, a synthetic component, a mixture of more than one natural component, a mixture of more than one synthetic component, a mixture of natural and synthetic components, or any combination thereof.

In another embodiment, the tensile element of the second tubular element comprises an organic or natural component, such as a fibrous protein, including without limitation, collagen, cellulose, silk, and keratin. Those of ordinary skill in the art will appreciate other natural fibrous proteins that may be suitable for use in the scaffolds of the present invention. In other embodiments, the tensile element is a synthetic component. Examples of synthetic tensile components, include without limitation, nylon, Dacron® (polyethylene terephthalate (PET)) Goretex® (polytetrafluoroethylene), polyester, polyglycolic acid (PGA), poly-lactic-co-glycolic acid (PLGA), and poly(etherurethane urea) (PEUU). In one embodiment, the present invention contemplates second tubular elements in which the tensile element includes a natural tensile component and a synthetic tensile component.

The elastomeric and tensile elements of the scaffolds may contain different combinations of natural and synthetic components. For example, a scaffold may contain a natural elastic component and/or a natural tensile component, and a synthetic elastic component and/or a synthetic tensile component.

In one aspect of the present disclosure, the TE scaffolds are not limited to a two layer structure having a second tubular element over a first tubular element, as described above. In some embodiments, the scaffolds include additional tubular elements, such as a third tubular element over the second tubular element, a fourth tubular element over the third tubular element, a fifth tubular element over the fourth tubular element, etc. In addition, as described herein, the additional tubular elements may contain an elastomeric element(s) (e.g. natural and/or synthetic) or a tensile element(s) (e.g. natural and/or synthetic). The additional tubular elements may be bonded by the techniques described herein.

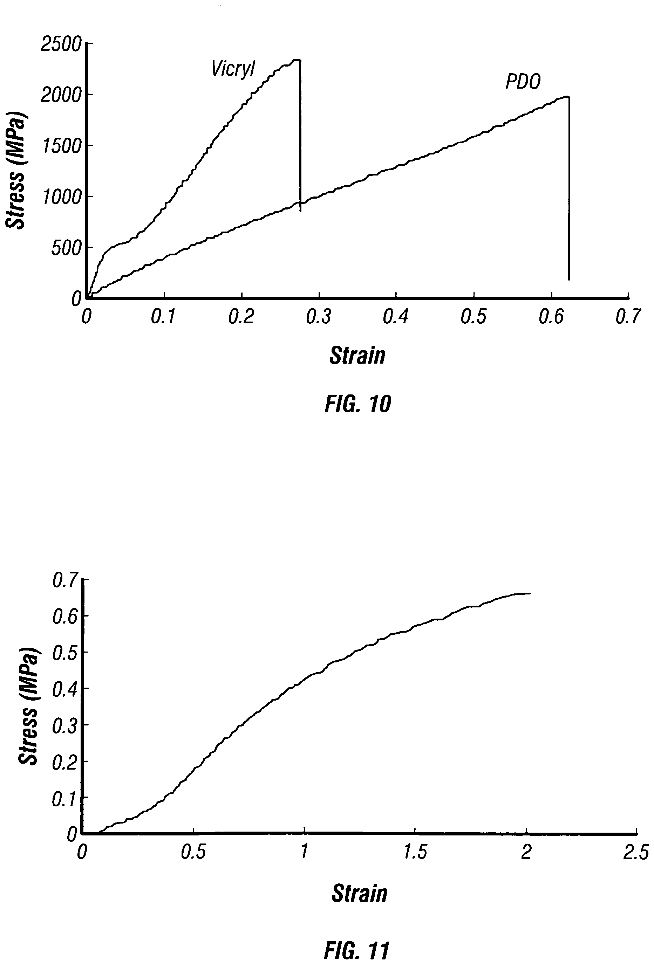

In one aspect, the elastomeric component contained in the elastomeric element and the tensile component contained in the tensile element each have a different elastic modulus. In one embodiment, the elastic modulus of the elastomeric component of the elastomeric element has a first elastic modulus and the tensile component of the tensile element has a second elastic modulus. In a preferred embodiment, the second elastic modulus is greater than the first elastic modulus by at least about one order of magnitude. In one embodiment, the second elastic modulus is greater than the first elastic modules by about one order of magnitude, about two orders of magnitude, about three orders of magnitude, about four orders of magnitude, or additional orders of magnitude. For instance, Example 1 shows the tensile components PDO and Vicryl to have elastic moduli of 3 GPa and 9-18 GPa, respectively, as compared to the 0.3 MPa to 0.5 MPa elastic modulus of the elastomeric component latex (see also Figures 10 and 11).

In another aspect, the TE scaffolds of the present disclosure exhibit structural and functional properties substantially similar to those found in native blood vessels. In native blood vessels, the synergistic interplay of two major protein components, collagen and elastin, gives rise to a mechanical response to stress and strain characterized by a J-shaped stress/strain curve (Roach et al. (1957) Can. J. Biochem. Physiol. 35:681-690). Those of ordinary skill in the art will appreciate the numerous parameters that can be used to demonstrate that the scaffolds of the present invention mimic or closely resemble native blood vessels, including without limitation, a response to stress and strain, compliance, Young's modulus, porosity, strength, etc. In one embodiment, the scaffolds of the present invention are characterized by having the ability to respond mechanically to stress and strain in an anisotropic manner.

A number of well-recognized parameters in the art are useful for characterizing the behavior of tissue engineering scaffolds. Table 1 provides examples of reported values (and their respective publication citation) for some of these parameters.

**Table 1**

| | **Parameter** | **Reported value(s)** |
|---|---|---|
| **Material** | Wall Thickness (µm) | 1000¹; 220-520²; 1000⁶; 1500¹²; 500-980²⁰; 50²³; 160-475²⁴; 1900-3800²⁸; 500-700²⁹; 1500-2500³³; 1000⁴⁴; |
| | Porosity (%) | 90²; 56,86⁶; 55-75¹⁰; 83-86¹¹; 80¹²; 93-95¹⁴; 62-81¹⁶; 90¹⁷; 97¹⁹; 80^{29;} 58-86³⁵; 91^{13,39}; 81-85⁴¹; |
| | Pore Diameter (µm) | 0.2-10⁴; 0.6-6⁵; 5-30³⁷; 120-50¹⁰; 100-200^{13,39}; 131-151^{2,14}; 200¹¹; 7-280³⁵; |
| | Fiber diameter (µm) | 0.1-4.5⁴; 0.4-1.2⁵; 0.1-0.2⁹;0.71-0.76¹¹; 0.18-1.4¹⁶; 0.22-0.6¹⁷; 13³⁹; 0.47-2.4²²; 12²⁶; 0.1-0.73³⁴; 0.22-0.88⁴¹; 0.37-107⁴⁴; |
| **Tube-Circumferential** | Breaking Strain (1/1) | 90-180²; 50-250⁵; 200-500⁶; 145⁷; 160-280⁸; 110-165⁹; 500-600¹⁰; 137-139¹²; 20-41¹³; 42-60¹⁶; 22-110²⁵; 110-190²⁷; 127³²; 150³³; 82-443³5; 150³⁶; |
| | Breaking Stress (MPa) | 0.01-0.03²; 3-6⁵; 3⁷; 2-13⁸; 0.25-2.35⁹; 3.39¹⁰; 22-24¹²; 0.06-0.24¹³; 3-17¹⁶; 0.02-0.05¹⁸; 0.4-0.7²¹; 0.15-0.83²²; 0.167²⁵; 1.5-3.7²⁷; 1.3-1.6³¹; 5.0³²; 1.5³⁴; 0.97-4.11³⁵; 0.043-0.101⁴⁰; 0.8-8.3⁴²; 1.15-7.55⁴³; |
| | Elastic Modulus 1 (MPa) | 0.002-0.004²; 0.22-0.28³⁶; 5-21⁵; 0.34-2.08²²; 1-8⁸; 0.51-0.92⁹; 1.22¹⁰; 0.048-.1⁴⁰; 1.5-2.5⁷; 45-57³⁴; |
| | Elastic Modulus 2 (MPa) | 0.003-0.015²; 1-8⁸; 9.1-61.9⁴³; |
| **Tube-Axial** | Breaking Strain (1/1) | 45-250¹⁶; |
| | Breaking Stress (MPa) | 1-5¹⁶; 0.7³⁴; |
| | Elastic Modulus 1 (MPa) | 20-30³⁴; |
| **Vessel** | Burst Pressure (mm-Hg) | 916-2347³; 1300¹⁵; 1775-3263²⁰; 2000-6000²⁴, 250-1150²⁵; 1200-3000²⁷; 1500-3000²⁸; 20-80³⁰; 74-90³³; 1327-3667⁴²; 697-3735⁴³; |
| | Compliance (%/100 mm-Hg) | 0.8-3.0³; 0.1-6⁵; 0.61³⁸; 3.3-22.8⁴³; |

| | | |
|---|---|---|
| 1) Burton AC: Physiol Rev 34:619,1954 2) Buttafoco L et al., Biomaterials 27:2380,2006 3) Smith MJ et al., Acta Biomat. 4:58,2007 4) Boland ED et al., Frontiers in Biosci. 9:1422,2004 5) Sell SA et al., Biomed Mater 1:72,2006 6) Jeong SI et al., J. Biomater Sci Polym Ed. 15:645,2004 7) Lim SH et al., J. Biomed. Mat. Res. B Epub ahead of print 2007 8) Stankus JJ et al., J Biomed Mat Res, 70A:63,2004 9) Barnes CP et al., Tiss Eng 13:1593, 2007 10) Kim SH et al., J Biomater Sci Polym Ed. 17:1359,2006 11) Nam J et al., Tiss Eng 13:2249, 2007 12) Watanabe M et al., Tiss Eng 7:429, 2001 13) Jeong SI et al., Biomaterials 28:1115,2007 14) Engbers-Nuijtenhuijs P et al., Biomaterials 27:2390, 2007 15) Amiel GE et al., Tiss Eng 12:2355, 2006 16) Boland ED et al., Acta Biomat 1:115, 2005 17) Buttafoco L et al., Biomaterials 27:724,2006 18) Cummings CL et al., Biomaterials 25:3699, 2004 19) Heydarkhan-Hagvall S et al., Tiss Eng 4:831, 2006 20) Hoerstrup et al., Eur J CardioThorac Surg 20:164,2001 21) Ishii Y et al., Ann Thorac Surg 83:517, 2007 22) Lee SJ et al., J Biomed Mater Res A 83:999,2007 23) Lepidi, S et al., FASEB J 20:103,2006 24) L'Heureux N et al., Nature Med 12:361, 2006 25) Lu Q et al., Biomaterials 25:5227,2004 26) Mooney DJ et al., Biomaterials 17:115, 1996 27) Nieponice A et al., Biomaterials Epub ahead of print 2007 28) Niklason LE et al., Science 284:489, 1999 29) Shinoka T et al., J. Thorac Card Surg 129:1330, 2005 30) Weinberg CB et al., Science 399, 1986 31) Wu H et al., Biomaterials 28:1385, 2007 32) Xu C et al., Tiss Eng 10:1160,2004 33) Aper T et al., Eur J Vasc Endovasc Surg 1,2006 34) Matthews JA et al., Biomacromolecules 3:232, 2002 35) Guan J et al., Cell Transplantation 15:S17, 2006 36) Mithieux SM et al., Biomaterials 25:4921,2004 37) Zhang Z et al., Biomaterials 25:177,2004 38) Solan A et al., Tiss Eng 9:579,2003 39) Jeong SI et al., Biomaterials 26:1405,2005 40) Hahn MS et al., Ann Biomed Eng 35:190, 2007 41) Boland et al., J Biomed Mater Res B: Appl Biomater 71B:144, 2004 42) Dahl SLM et al., Cell Transplant 12:659,2003 43) Dahl SLM et al., Ann Biomed Eng 35:348,2007 44) Stitzel J et al., Biomaterials 27:1088, 2006 | | |

Table 2 provides characterization specifications based upon the literature cited in Table 1 that project to provide mechanical properties to a TE scaffold or TEBV that are substantially similar to a native blood vessel.

**Table 2**

| **Test** | **Parameter** | **Value** |
|---|---|---|
| **Material** | Wall Thickness (µm) | 600 - 1200 |
| | Porosity (%) | 90 - 99 |
| | Pore Diameter (µm) | 5 - 100 |
| | Pore Gradient (µm) | Adventitial side pore size ∼100µm to luminal pore size o ∼5µm to ∼15µm. |
| | Fiber diameter (µm) | 0.05 - 20 |
| **Tube-Circumferential** | Breaking Strain (1/1) | 1.1 - 1.5 |
| | Breaking Stress (MPa) | 1.5 - 3.5 |
| | Elastic Modulus 1 (MPa) | 0.1 - 0.5 |
| | Elastic Modulus 2 (MPa) | 3.0-6.0 |
| | Modulus 1 to Modulus 2 Transition | 0.57 - 1.12 |
| | Toughness (MJ/m³) | 0.45 - 1.0 |
| **Tube-Axial** | Breaking Strain (1/1) | >0.8 |
| | Breaking Stress (MPa) | >0.75 |
| | Elastic Modulus 1 (MPa) | 0.1 - 0.3 |
| | Elastic Modulus 2 (MPa) | 1.0 - 6.0 |
| | Modulus 1 to Modulus 2 Transition | 0.64 - 0.80 |
| | Toughness (MJ/m³) | 0.1 - 0.5 |
| **Tube**-**Viscoelastic Properties** | Tan Delta | 0.05 - 0.3 |
| | Storage Modulus (MPa) | 400 - 0.12 |
| **Vessel** | Burst Pressure (mm-Hg) | 1300-2000 |
| | Compliance (%/100 mm-Hg) | 2.5 - 5.0 |
| | Kink Radius (mm) | 5 - 12 |

These parameters are useful in characterizing the mechanical behavior of a tissue engineering scaffold of the present invention, and in particular, in determining whether the scaffold will exhibit properties substantially similar to that of a native blood vessel. The present disclosure is directed to tissue engineering scaffolds that are characterized by the values of Table 2 and that exhibit mechanical properties substantially similar to those of a native blood vessel, preferably (i) a mechanical response to stress and strain characterized by a J-shaped stress/strain curve; (ii) resistance to fracturing; (iii) viscoelasticity; or (iv) any combination of (i)-(iii). In addition, the scaffolds are characterized by accessibility to various cell types for the purpose of cell seeding to form a TEBV.

In one embodiment, the characteristic of a J-shaped stress/strain curve exhibited by the tissue engineering scaffolds is attributable to (i) a circumferential tube elastic modulus 1 of about 0.1 MPa to about 0.5 MPa, (ii) a circumferential tube elastic modulus 2 of about 3.0 MPa to about 6.0 MPa; and (iii) a circumferential modulus transition of about 0.57 to about 1.12, and any combination thereof. In another embodiment, the circumferential tube elastic modulus 1 is about 0.1 MPa, 0.13 MPa, about 0.15 MPa, about 0.17 MPa, about 0.2 MPa, about 0.22 MPa, about 0.25 MPa, about 0.27 MPa, about 0.3 MPa, about 0.32 MPa, about 0.35 MPa, about 0.37 MPa, about 0.4 MPa, about 0.42 MPa, about 0.45 MPa, about 0.47 MPa, or about 0.5 MPa. In another embodiment, the circumferential tube elastic modulus 2 is about 3.0 MPa, about 3.2 MPa, about 3.5 MPa, about 3.7 MPa, about 4.0 MPa, about 4.2 MPa, about 4.5 MPa, about 4.7 MPa, about 5.0 MPa, about 5.2 MPa, about 5.5 MPa, about 5.7 MPa, or about 6.0 MPa. In another embodiment, the circumferential modulus transition is about 0.57, about 0.59, about 0.61, about 0.63, about 0.65, about 0.67, about 0.69, about 0.71, about 0.73, about 0.75, about 0.77, about 0.79, about 0.81, about 0.83, about 0.85, about 0.87, about 0.89, about 0.91, about 0.93, about 0.95, about 0.97, about 0.99, about 1.01, about 1.03, about 1.05, about 1.07, about 1.09, about 1.11, or about 1.12.

In another embodiment, the property favoring resistance to fracture is (i) a circumferential tube toughness of about 0.45 MJ/m³ to about 1.0 MJ/m³; (ii) an axial tube toughness of about 0.1 MJ/m³ to about 0.5 MJ/m³; or (iii) a combination of (i) and (ii). The toughness of a biomaterial is one parameter that helps determine its resistance to fracture. Clearly, the resistance to fracturing or tearing is a desired feature in a TE scaffold because it helps ensure the patency of any TEBV or vascular graft derived therefrom. Native blood vessels are subject to deformation in response to the stress and strain of cyclic loading of fluid. As such, they are at risk for a split or fracture in a longitudinal or axial manner and/or a circumferential manner. Similar to native blood vessels, the vascular grafts derived from the TE scaffolds and TEBVs are also at risk for a fracture. The present invention concerns the discovery that a particular axial toughness and/or a particular circumferential toughness contributes to a TE scaffold that is resistant to fracture or tearing. In one embodiment, the circumferential tube toughness is about 0.45 MJ/m³, about 0.50 MJ/m³, about 0.55 MJ/m³, about 0.60 MJ/m³, about 0.65 MJ/m³, about 0.70 MJ/m³, about 0.75 MJ/m³, about 0.80 MJ/m³, about 0.85 MJ/m³, about 0.90 MJ/m³, about 0.95 MJ/m³, about 1.0 MJ/m³. In another embodiment, the axial tube toughness is about 0.1 MJ/m³ about 0.15 MJ/m³, about 0.20 MJ/m³, about 0.25 MJ/m³, about 0.30 MJ/m³, about 0.35 MJ/m³, about 0.40 MJ/m³, about 0.45 MJ/m³, or about 0.50 MJ/m³. In another embodiment, the TE scaffolds are characterized by one or more of: i) a scaffold which has a mechanical response to stress and strain characterized by a J-shaped stress/strain curve; ii) a fracture-resistant scaffold; and iii) a viscoelastic scaffold.

In another embodiment, the viscoelastic properties of a TE scaffold are characterized by (i) a tangent delta of about 0.05 to about 0.3; (ii) a storage modulus of about 400 MPa to about 0.12 MPa; or (iii) a combination of (i) and (ii). Viscoelastic materials exhibit both viscous and elastic characteristics in response to deformation. While viscous materials resist strain linearly with time when stress is applied, elastic materials strain instantly in response to stress and rapidly return to their original state once the stress is removed. A viscoelastic material exhibits a time-dependent strain in response to stress, which typically involves the diffusion of atoms or molecules within an amorphous material. As native blood vessels display viscoelasticity to cope with the cyclic loading of fluid, this trait is desirable for the TE scaffolds that will be used to create a TEBV or vascular graft. The present invention concerns the discovery that the viscoelasticity of a TE scaffold of the present invention is characterized by a particular tangent delta value and/or a particular storage modulus value. In one embodiment, the tangent delta is about 0.05, about 0.06, about 0.07, about 0.08, about 0.09, about 0.10, about 0.11, about 0.12, about 0.13, about 0.14, about 0.15, about 0.16, about 0.17, about 0.18, about 0.19, about 0.20, about 0.21, about 0.22, about 0.23, about 0.24, about 0.25, about 0.26, about 0.27, about 0.28, about 0.29, or about 0.30. In other embodiments, the storage modulus is about 400 MPa, about 350 MPa, about 300 MPa, about 250 MPa, about 200 MPa, about 150 MPa, about 100 MPa, about 90 MPa, about 80 MPa, about 70 MPa, about 60 MPa, about 50 MPa, about 40 MPa, about 30 MPa, about 20 MPa, about 10 MPa, about 9 MPa, about 8 MPa, about 7 MPa, about 6 MPa, about 5 MPa, about 4 MPa, about 3 MPa, about 2 MPa, about 1 MPa, about 0.9 MPa, about 0.8 MPa, about 0.7 MPa, about 0.6 MPa, about 0.5 MPa, about 0.4 MPa, about 0.3 MPa, about 0.2 MPa, about 0.19 MPa, about 0.18 MPa, about 0.17 MPa, about 0.16 MPa, about 0.15 MPa, about 0.14 MPa, about 0.13 MPa, or about 0.12 MPa.

There are several techniques well-known to those of ordinary skill in the art that are suitable for identifying and characterizing the desirable properties for the scaffolds of the present invention. These techniques include, without limitation, burst pressure testing; quasi-static mechanical testing (a.k.a. tensile testing) in the circumferential direction (results provided in a stress/strain diagram); determining porosity and pore size (e.g. by mercury intrusion porosimetry); cell attachment assays; and degradation rate; pressure/volume curves for measurement of graft compliance.

### 4. Methods of making TE scaffolds

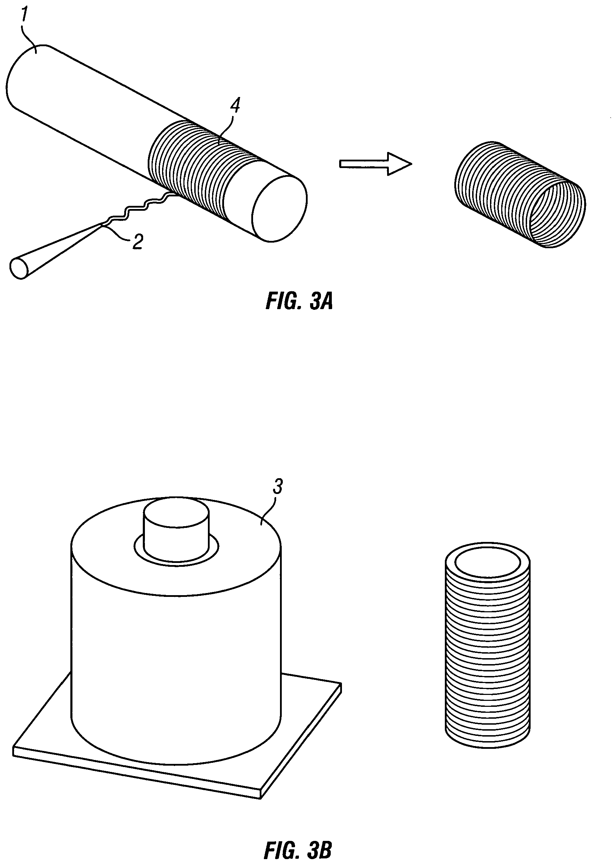

The methods disclosed herein concern the construction of TE scaffolds that possess suitable, long-lasting biomechanical properties commensurate with native blood vessels. In one aspect, the methods provide methods of making vessel scaffolds having a laminated structure, namely a first tubular element comprising an elastomeric element, an exterior surface and an interior luminal surface; and a second tubular element comprising a tensile element, an exterior surface and an interior luminal surface in contact with the exterior surface of the first tubular element. As illustrated in Figure 3, the first tubular element (4) may be formed on a mandrel (1) by techniques known in the art, including without limitation, electrospinning (2) (Figure 3A) and casting (3) (Figure 3B), and any combination thereof. An elastomeric element, such as elastin and/or an elastomeric polymer, may be used to form the first tubular element having a first diameter, which is at least the nominal size needed for an *in vivo* application. Electrospinning may be performed by applying solutions (i) containing one or more elastomeric natural components and/or one of more elastomeric synthetic components; and/or (ii) containing one or more tensile natural components and/or one of more tensile synthetic components. Electrospinning offers the benefit of circumferential arrangement of the fibers of the elastomeric element, thus increasing the strength of the vessel.

Once formed, the first tubular element containing an elastomeric element is dilated to a second diameter by techniques known in the art, including without limitation, utilizing a mandrel with a variable diameter, or removal and placement of the first tubular element on a larger mandrel. The use of a mandrel with a variable diameter has the advantage of avoiding the removal of the first tubular element from the mandrel which can be problematic due to friction. Dilation to the second diameter of the first tubular element is intended to account for the extent of physiological strains that arteries are subjected to during normal function, i.e. about 5% to about 35%.

In one embodiment, the first tubular element is formed by a technique described herein to have a first diameter of about 1 mm, about 2 mm, about 3 mm, about 4 mm, about 5 mm, about 6 mm, about 7 mm, about 8 mm, about 9 mm, or about 10 mm. In a preferred embodiment the first diameter is from about 3 mm to about 8 mm, more preferably from about 4 mm to about 7 mm, and most preferably from about 5 mm to about 6 mm.

In another embodiment, the second diameter to which the first tubular element is dilated to by a technique described herein is about 4 mm, about 5 mm, about 6 mm, about 7 mm, about 8 mm, about 9 mm, about 10 mm, about 11 mm, about 12 mm, about 13 mm, about 14 mm, about 15 mm, or about 16 mm. In a preferred embodiment the second diameter is from about 5 mm to about 10 mm, more preferably from about 6 mm to about 9 mm, and most preferably from about 7 mm to about 8 mm.

Once dilated to the second diameter, a second tubular element is formed or layered on the exterior surface of the first tubular element by techniques known in the art, including without limitation, casting or electrospinning. A tensile element, such as collagen and/or a tensile polymer, may be used to form the second tubular element. Electrospinning as a method of providing or forming the second tubular element is advantageous due to its ability to form tensile fibers of varying lengths.

Following the formation of the second tubular element over the first tubular element, the layers can be bonded by various techniques known to those of ordinary skill in the art. Such techniques include, without limitation, the use of a surgical adhesive such as one based on fibrin; or the use of a solvent interaction with the proportions of any synthetic polymers that are present.

In one embodiment, the bonding step is performed after the second tubular element is formed or placed over the first tubular element and includes the step of applying an additional layer of material on the outer surface of the second tubular element to allow adhesion sandwiching of the second tubular element between the first tubular element and the additional layer of material. In another embodiment, the additional layer contains the same type of material that was used to form the first tubular layer. In another embodiment, the bonding is achieved via electrospinning application of an additional layer containing an elastomeric element (e.g. a Solution 1 containing, for example, a natural/synthetic elastomeric material mix as described below).

Those of ordinary skill in the art will appreciate that other techniques can be used to crosslink both within and among the layers. For example, thermal treatment has been shown to form crosslinks in a tensile element (e.g. collagen) based on condensation reactions. Other biocompatible chemical crosslinking treatments have been shown to be effective in this area as well.

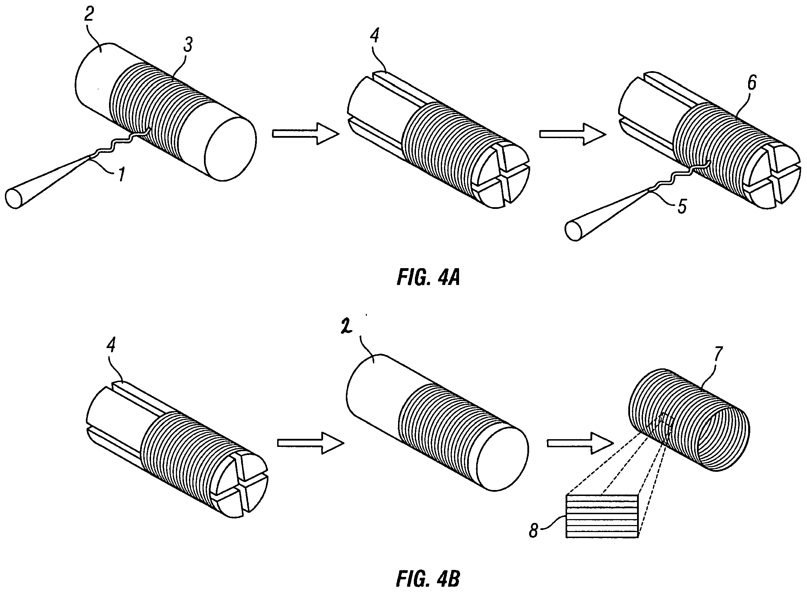

Figure 4A exemplifies the electrospinning method of creating the novel scaffold architectures described herein. Solution 1 (1) containing, for example, a natural/synthetic elastomeric material mix is spun on a rotating mandrel (2) to create a first tubular element (3) having a first diameter of Dₒ. Then, the mandrel's diameter is increased (4) (alternatively, the scaffold is placed on a mandrel of larger diameter) to a second diameter D_{f}, a value commensurate with physiological strains in native vessels, and Solution 2 (5) containing, for example, a natural/synthetic tensile material mix is electrospun onto the first tubular element created with Solution 1 to form a second tubular element (6) over the first tubular element having a second diameter of D_{f}. This results in the formation of a pre-stressed laminate structure. The final step shown in Figure 4B involves returning the mandrel to the first diameter, Do (2), and removing the scaffold (7) that now includes both the first and second tubular elements. Structural analysis of the outer laminate made up of Solution 2 will reveal corrugations (8) in the fibrous structure in a circumferential direction, shown in the blown-up portion of Figure 4B.

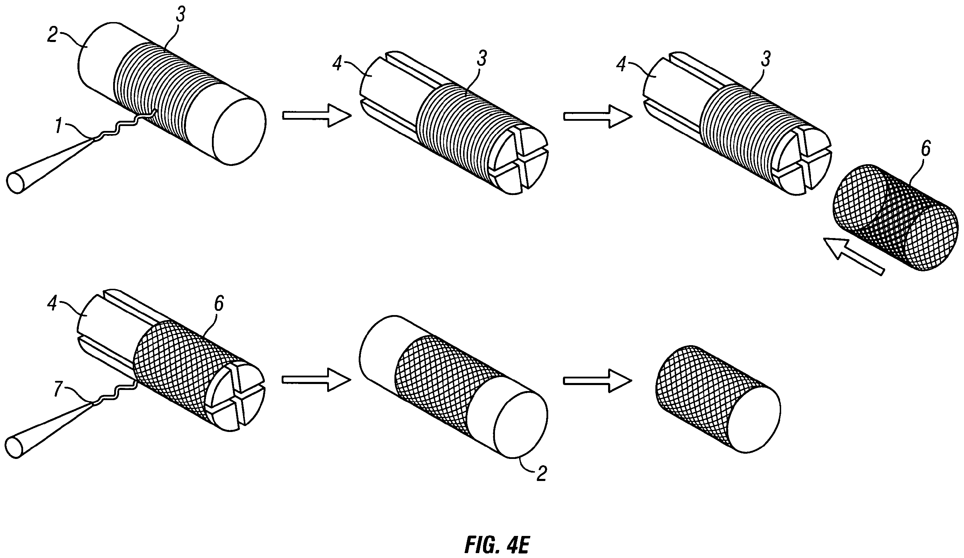

Figure 4E depicts an alternative embodiment, of the expanding mandrel process. A first tubular element is formed from Solution 1 (1), as described above, to create a first tubular element (3) having a first diameter of Dₒ (2). The diameter of the mandrel is increased to a second diameter D_{f} (4), at which point a second tubular element (6) containing natural/synthetic tensile material is placed over the first tubular element while it is on the expanded mandrel. After placement of the second tubular element, an additional thin layer of Solution 1 (7) may be electrospun on top of the second tubular element to allow adhesion sandwiching of the second tubular element in between the respective layers of Solution 1. Following application of the thin layer of Solution 1, the diameter of the expanded mandrel is returned to the first diameter of Dₒ (2). The contraction of the first tubular element entrains the second tubular element causing a corrugated or kinked uniform surface feature. In one embodiment, the second tubular element is in the form of a mesh.

As described above, it is well known that compliance mismatch at the interface where a vascular graft is joined to a native blood vessel can lead to intimal hyperplasia, which is an important failure mode associated with loss of patency in vascular grafts. It is known that such intimal hyperplasia can lead to aneurysm formation and dilatation of the graft. A problem that arises in multi-laminate structures where the respective laminae possess differing compliances is delamination. This can be a particular problem in structures where there is a sudden transition among laminae and thus potential for correspondingly high stress concentrations. To combat this, the sudden transition among laminae can be lessened by ensuring that each successive layer possesses a zone of heterogeneity where it impinges on the next successive zone.

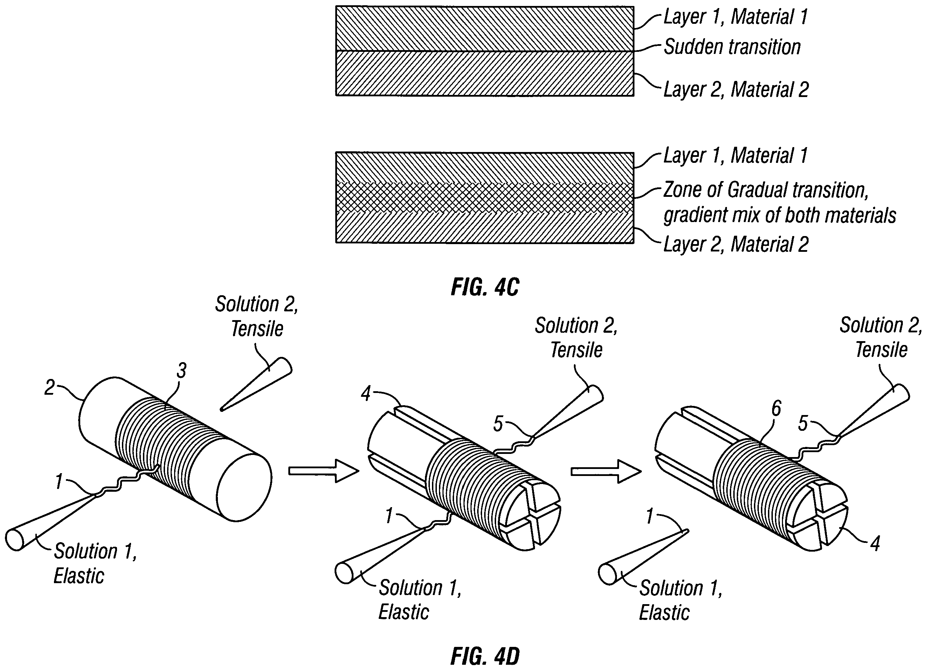

Figure 4C illustrates this concept. As a transition between layers is approached, a gradual gradient exists upon mixing between the two materials that comprise the neighboring layers. This zonal gradation can be accomplished in a number of ways, but most apparently through the use of multiple syringes and solution gradients.

Figure 4D illustrates an electrospinning methodology for achieving zonal gradation. The method employs two spinnerets, two material solutions, and sequential application (with overlap) in order to generate a gradual transition between the lamina constructed from the two materials. Solution 1 (1) containing, for example, a natural/synthetic elastomeric material mix is spun onto a rotating mandrel (2) to create a first tubular element (3) having a first diameter of Dₒ but before the application of Solution 1 is complete, the mandrel is expanded to a second diameter of D_{f} (4) and Solution 2 (5) containing, for example, a natural/synthetic tensile material mix is applied to form a second tubular element (6) over the first tubular element. Preferably, the application of Solution 2 is begun close to the end of the application of Solution 1. The application of Solution 1 and Solution 2 continues simultaneously until the completion of Solution 1's application. Thus, a gradual blending of the material in Solution 1 and the material in Solution 2 is created at the zone between the first and the second tubular elements. This results in the formation of a pre-stressed laminate structure having zonal gradation.

In one embodiment, a tissue engineered scaffold of the present disclosure that comprises a zonal gradation is made up of a first tubular element containing an elastomeric element, a second tubular element containing a tensile element contacted with the exterior of the first tubular element, and a zone of gradual transition or gradient mix of the elastomeric element in the first tubular element and the tensile element of the second tubular element. In another embodiment, the tissue engineered scaffold's zonal gradation contains a transitional zone of heterogeneity having materials from each of the first and second tubular elements.

As described above, the present disclosure provides methods in which the diameter of a mandrel is increased from a first diameter (Dₒ) to a discrete second diameter (D_{f}) and then subsequently returned to the first diameter (Dₒ). In another embodiment, the present disclosure provides methods in which the mandrel diameter is increased from a first diameter (Dₒ) to a second diameter (D_{f}) over a continuum of diameter increases during electrospinning. To achieve a continuum of diameter increases, a mandrel may be programmed such that the diameter increases from a first diameter (Dₒ) to a second diameter (D_{f}) at a continuous rate during the electrospinning of Solution 2 to form a second tubular element over the first tubular element. This approach would ensure that during stretching of the two-layer laminar structure in the circumferential direction, the outer tensile layer would engage at different strain values and ensure a more gradual curvature associated with a more natural "J" shaped curve.

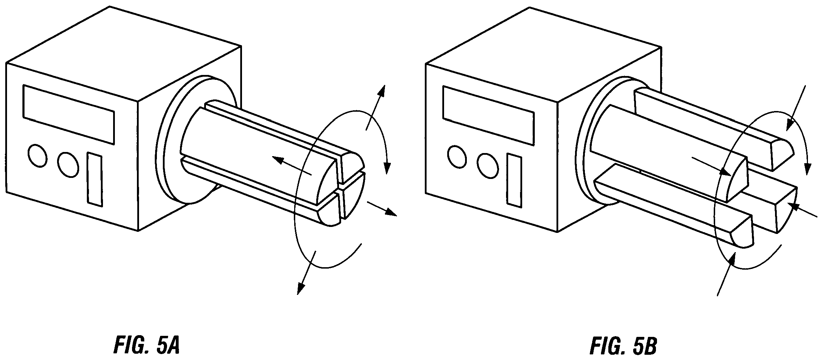

Figure 5 illustrates an expanding mandrel device capable of continuous diameter change during rotation. Figure 5A shows a near minimum diameter at which an electrospun tube could be easily removed by contracting the mandrel's diameter. Figure 5B shows the mandrel in a maximum diameter configuration. The mandrel sections are in screw-driven tracks that allow continuous movement during spinning at a preprogrammed rate.

In one embodiment, the steps of increasing and decreasing the diameter of a novel scaffold architecture are performed in parallel with an electrospining step. In a preferred embodiment, the first diameter Dₒ of a first tubular element formed from a Solution 1 containing, for example, a natural/synthetic elastomeric material mix is increased to the second diameter D_{f} at a steady continuous rate while simultaneously electrospinning a Solution 2 containing, for example, a natural/synthetic tensile material mix onto the first tubular element. The end result is the deposition of a second tubular element containing a tensile element onto the first tubular element containing an elastic element such that the tensile element exists in the novel scaffold architecture as a continuum of tensile fibers. Upon the cycling of gradually increasing volumes of fluid through the novel scaffold architecture, the first diameter Dₒ of the first tubular element will in response gradually increase due to the elastic element contained in the first tubular element, and as it does so, the continuum of tensile fibers present in the second tubular element will engage over the continuum as the first diameter Dₒ gradually increases to the second diameter D_{f} due to the cycling of fluid. As such, the continuum of tensile fibers created by the methods instills in a novel scaffold architecture the property of gradual engagement of tensile fibers as the volume of fluid passing through increases. Such a property further contributes to the substantial similarity to native blood vessels exhibited by the tissue engineering scaffolds of the present disclosure.

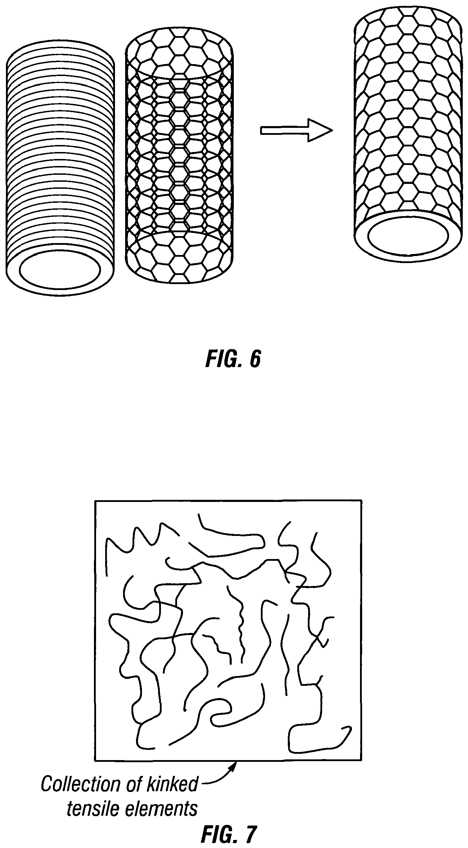

As described above, the methods may employ electrospinning for the creation of a second tubular element over a first tubular element. In one embodiment, the second tubular element is not formed by electrospinning but rather is a knitted, woven or mesh structure of monofilament (one filament thick in the radial direction) that may be placed over the first tubular element. In this embodiment, the first tubular element is created and expanded to the desired diameter with a chosen strain value before maneuvering the knitted/woven/mesh second tubular element into place surrounding the first tubular element. The size of the knitted/woven/mesh second tubular element may be preselected in order to fit snugly with the first tubular element at the desired expansion size as shown in Figure 6. Following placement, the second tubular element may be fastened to the first tubular element through the bonding techniques described above.

As described herein, the blood vessel scaffold includes a first tubular element comprising an exterior surface and an interior luminal surface, and a second tubular element comprising an exterior surface and an interior luminal surface. Following bonding, the exterior surface of the first tubular element is in contact with the interior luminal surface of the second tubular element. The interior luminal surface of the first tubular element and the exterior surface of the second tubular element are both accessible at this point for further manipulation.

After bonding is complete, the second diameter of the first tubular element is decreased to its first and original diameter. This may be performed by reducing a variable mandrel to the first diameter or, in the case of casting, simply by removing the scaffold from a larger mandrel. The constriction of the first tubular element back to its first diameter imparts a series of corrugations to the fibers of the second tubular element containing a tensile element.

In one other embodiment, the methods include the application of fibers to a first tubular element (containing an elastomeric element) with variable degrees of kink, in particular tensile filaments having an inherent degree of kinking. Such fibers can, for example, be isolated from a non-woven felt such is used for the formation of bladder replacement scaffolds. These fibers are 12-18 µm in diameter (length: ~2cm) and have a kinked morphology based on a prerequisite need for this geometry for the needling process in nonwoven felt formation.

Figure 7 illustrates fiber morphologies that can be realized from felt materials. The varying morphologies contribute to a continuum of stiffening as a first tubular element (containing an elastomeric element) is expanded. In one embodiment, these non-continuous fibers are adhered to the first tubular element at its desired expanded size, and optionally, are generally oriented in the circumferential direction. Once applied, the fibers can be sealed in or bound to the first tubular element by the application of one of the bonding techniques described above. In another embodiment, a fiber-to-fiber linkage is provided within the material itself to impart a continuum of stiffening based on the variety of different degrees of individual fiber morphology, as illustrated in Figure 7. This fiber-to-fiber linkage may be performed prior to bonding. Mechanically, the advantageous effect of the application of these fibers (once linked and/or bonded to the first tubular element) is that upon strain, the fibers with the least amount of kinking will straighten first, and engage. Since there is a continuum in the degree of kinking in the fibers applied, as strain increases, fibers will engage at varying intervals leading to a gradual rounding of the stress/strain diagram thus providing a response much more akin to native materials.

In a preferred embodiment, the method of making a tissue engineered blood vessel scaffold comprises the steps of (a) providing or forming an elastomeric tubular element comprising an exterior surface, an interior luminal surface, and a first diameter; (b) dilating the elastomeric tubular element to a second diameter; (c) providing or forming a tensile tubular element comprising an exterior surface and a second diameter on the exterior surface of the elastomeric tubular element of step (b); (d) bonding the tensile tubular element to the exterior surface of the elastomeric tubular element; and (e) decreasing the second diameter of the elastomeric tubular element to the first diameter.

In one aspect, the methods provided herein allow a person of ordinary skill in the art to exercise a high degree of tunability making the TE scaffolds. By varying different aspects of the methods, the mechanical properties described herein are subject to tuning in the manner desired by the skilled artisan. In one embodiment, the tuning of mechanical properties comprises alteration of one or more of the following: the choice of materials used to provide the tubular scaffolds, the diameter of expansion of a tubular element, the distance between the needle and the mandrel during electrospinning, and the thickness of the the tubular elements employed. In another embodiment, the tuning comprises alteration of one or more parameters listed in Table 2 above. Those of skill in the art will appreciate other parameters that may be altered to tune the mechanical properties of the TE scaffolds.

### 5. Tissue Engineered Blood Vessels (TEBVs)

In another aspect, the present disclosure provides tissue engineered blood vessels (TEBVs) that are derived from the TE scaffolds of the present invention. Given their substantial similarity to native blood vessels, the scaffolds are particularly amenable to modification to create TEBVs that in turn can be used as vascular bypass grafts for the treatment of cardiovascular disorders. Vascular bypass grafts include arteriovenous (AV) shunts. In a preferred embodiment, the scaffolds of the present invention can be used to create TEBVs having a small diameter, typically less than 6 mm, for use in treating cardiovascular disorders.

As discussed herein, certain embodiments of the TE scaffolds have been shown to exhibit a mechanical response to stress and strain characterized by a J-shaped stress/strain curve that is attributable to a range of elastic moduli and modulus transition, and any combination thereof. In addition to the moduli parameters, there are other properties exhibited by the TE scaffolds that make them attractive for use in making vascular grafts. In one aspect, the TE scaffolds of the present invention exhibit certain properties which render them particularly suitable for making a TEBV or vascular graft in the first place, and for ensuring that the vascular graft will retain patency once implanted. Such properties include, without limitation, those that allow the seeding of cells on a scaffold, those that provide resistance to fracture of the scaffold, and those that provide viscoelasticity to a scaffold.

In one embodiment, the property favoring the seeding of cells on the TE scaffolds is attributable to a pore gradient where the pore diameter gradually decreases from about 100 microns at the adventitial or exterior side to about 5 to about 15 microns at the luminal or interior side of a tubular element. It is well known in the art that pore diameter is an important factor for the successful seeding of cells on and within a TE scaffold. For example, the pore diameter must be large enough for various cell types to migrate to the surface of a scaffold and through a scaffold, such that they can interact with other migrating cells in a manner similar to that observed *in vivo.* The present invention concerns the discovery that a particular pore gradient contributes to the successful seeding of cells. In one embodiment, the pore gradient renders the TE scaffold accessible to cells and thereby enhances its capacity for cell seeding. In another embodiment, the pore gradient is about 100 microns (exterior side) to about 5 microns (interior side), about 100 microns (exterior side) to about 6 microns (interior side), about 100 microns (exterior side) to about 7 microns (interior side), about 100 microns (exterior side) to about 8 microns (interior side), about 100 microns (exterior side) to about 9 microns (interior side), or about 100 microns (exterior side) to about 10 microns (interior side).

In one aspect, the pore gradient provides architecture that is advantageous for the seeding of cells on the luminal, interior side of a TE scaffold and for the seeding of cells on the exterior, adventitial side of a TE scaffold. In one embodiment, the smaller pore size on the luminal, interior surface is suited for seeding of endothelial cells on and within the interior surface, and the larger pore size on the exterior, adventitial side is suited for seeding of smooth muscle cells on and within the exterior surface. In another embodiment, the endothelial cells are seeded to form a monolayer or flat sheet-like structure on and within the interior, luminal surface of the TE scaffold and/or the smooth muscle cells are seeded on and/or within the exterior, adventitial surface of the TE scaffold.

In some embodiments, the endothelial cells seeded on and throughout the interior, luminal surface of the TE scaffold are unable to migrate towards the exterior, adventitial surface beyond certain pore size. In a preferred embodiment, the pore size is about 15 to about 20 microns. In another preferred embodiment, the pore size is about 15 microns, about 16 microns, about 17 microns, about 18 microns, about 19 microns, or about 20 microns.

In another embodiment, the property favoring resistance to fracture is (i) a circumferential tube toughness of about 0.45 MJ/m³ to about 1.0 MJ/m³; (ii) an axial tube toughness of about 0.1 MJ/m³ to about 0.5 MJ/m³; or (iii) a combination of (i) and (ii). The toughness of a biomaterial is one parameter that helps determine its resistance to fracture.

In another embodiment, the property favoring the viscoelasticity of a TE scaffold is (i) a tangent delta of about 0.05 to about 0.3; (ii) a storage modulus of about 400 MPa to about 0.12 MPa; or (iii) a combination of (i) and (ii).

In another aspect, the present disclosure provides tissue engineered blood vessels (TEBVs) that are derived from the TE scaffolds described herein. As a result, the TEBVs exhibit structural and functional properties substantially similar to those found in native blood vessels. As discussed above, the synergistic interplay of two major protein components, collagen and elastin, in blood vessels gives rise to a mechanical response to stress and strain characterized by a J-shaped stress/strain curve (Roach et al. (1957) Can. J. Biochem. Physiol. 35:681-690). In one embodiment, the TEBVs of the present invention are characterized by having the ability to respond mechanically to stress and strain in an anisotropic manner. In another embodiment, the TEBVs have (i) properties favoring resistance to fracture of the scaffold; and/or (ii) properties favoring the viscoelasticity of a scaffold.

In another aspect, the tissue engineered blood vessels (TEBVs) can modulate certain complications associated with vascular grafts that have been observed following implantation. In one embodiment, the TEBVs modulate compliance mismatch after implantation. In another embodiment, the modulation comprises one or more of the following: resistance to aneurysm formation, resistance to dilatation, resistance to fracture, resistance to thrombosis, resistance to anastomotic hyperplasia, and resistance to intimal hyperplasia. Those of skill in the art will appreciate additional factors subject to modulation by the TEBVs.

In one embodiment, the a TEBV of the present invention comprises a TE scaffold as described herein. A TE scaffold may be further manipulated to form a TEBV that will be suitable for transplantation into a mammal in need. For example, the TE scaffold may be manipulated by adding one or more cell populations by the methods described herein. Those of ordinary skill in the art will appreciate that the present invention pertains to many types of blood vessels, including without limitation, the carotid artery, the subclavian artery, the celiac trunk, the mesenteric artery, the renal artery, the iliac artery, arterioles, capillaries, venules, the subclavian vein, the jugular vein, the renal vein, the iliac vein, the venae cavae.

In one embodiment, the TEBV further comprises a first cell population within the second tubular element and/or on the exterior surface of second tubular element of the TEBV. In a preferred embodiment, the first cell population is a smooth muscle cell population. Those of skill in the art will appreciate that various types of smooth muscle cells (SMCs) may be suitable for use (see Bertram et al. U.S. Published Application 20070190037 incorporated herein by reference in its entirety), including without limitation, human aortic smooth muscle cells, human umbilical artery smooth muscle cells , human pulmonary artery smooth muscle cells, human coronary artery smooth muscle cells , human bronchial smooth muscle cells, human radial artery smooth muscle cells ,and human saphenous or jugular vein smooth muscle cells. As described in Bertram et al. U.S. Published Application 20070190037, the SMCs may be isolated from a variety of sources, including, for example, biopsies from living subjects and whole-organ recover from cadavers. The isolated cells are preferably autologous cells, obtained by biopsy from the subject intended to be the recipient.

In another embodiment, the TEBV comprises a second cell population on the interior or luminal surface of the TEBV. In a preferred embodiment, the second cell population is an endothelial cell population. Those of skill in the art will appreciate that various types of endothelial cells (ECs) may be suitable for use (see U.S. Published Application 20070190037), including without limitation, arterial and venous ECs such as human coronary artery endothelial cells, human aortic endothelial cells, human pulmonary artery endothelial cells, dermal microvascular endothelial cells, human umbilical vein endothelial cells, human umbilical artery endothelial cells, human saphenous vein endothelial cells, human jugular vein endothelial cells, human radial artery endothelial cells, and human internal mammary artery endothelial cells. ECs may be isolated from a variety of sources including, without limitation, the vascular parenchyma, circulating endothelial cells and endothelial cell precursors such as bone marrow progenitor cells, peripheral blood stem cells and embryonic stem cells (see Bischoff et al. U.S. Published Application 20040044403 and Raffi et al. U.S. Patent 6,852,533).

Those of skill in the art will appreciate that the seeding or deposition of one or more cell populations described herein may be achieved by various methods known in the art. For example, bioreactor incubation and culturing, (Bertram et al. U.S. Published Application 20070276507; McAllister et al. U.S. Patent 7,112,218; Auger et al. U.S. Patent 5,618,718; Niklason et al. U.S. Patent 6,537,567); pressure-induced seeding (Torigoe et al. (2007) Cell Transplant., 16(7):729-39; Wang et al. (2006) Biomaterials. May;27(13):2738-46); and electrostatic seeding (Bowlin et al. U.S. Patent No. 5,723,324) may be used. In addition, a recent technique that simultaneously coats electrospun fibers with an aerosol of cells may be suitable for seeding or deposition (Stankus et al. (2007) Biomaterials, 28:2738-2746).

In one embodiment, the deposition of cells includes the step of contacting a tubular scaffold with a cell attachment enhancing protein. In another embodiment, the enhancing protein is one or more of the following: fibronection, collagen, and MATRIGEL™. In one other embodiment, the tubular scaffold is free of a cell attachment enhancing protein. In another embodiment, the deposition of cells includes the step of culturing after contacting a tubular scaffold with one or more cell populations. In yet another embodiment, the culturing may include conditioning by pulsatile and/or steady flow in a bioreactor.

In one aspect, the present disclosure provides methods of treating a cardiovascular disease or disorder in a subject in need thereof. In one embodiment, the method includes the step of identifying a subject in need. In another embodiment, the method includes the step of obtaining one or more biopsy samples from the subject. In one other embodiment, the method includes the step of isolating one or more cell populations from the sample and culturing the one or more cell populations on a TE scaffold to provide a TEBV. In another embodiment, the culturing includes conditioning of cell-seeded TEBV scaffold in a bioreactor. In one embodiment, the conditioning comprises steady and/or pulsatile flow in a bioreactor. In another embodiment, the method includes the implantation of the cell-seeded, conditioned TEBV into the subject in need to treat the cardiovascular disease or disorder.

Those of ordinary skill in the art will appreciate the various cardiovascular disorders that are suitable for treatment by the methods of the present disclosure.

In another embodiment, the present disclosure provides the use of the TE scaffolds and/or TEBVs described herein for the preparation of a medicament useful in the treatment of a cardiovascular disorder in a subject in need.

The following examples are offered for illustrative purposes only, and are not intended to limit the scope of the present invention in any way.

### EXAMPLES

### EXAMPLE 1 - Suture wrap over latex tubing

Generation of a "J"-shaped mechanical response in a two-component tubular architecture.

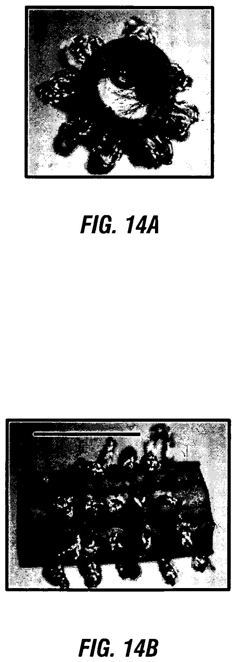

There are several ways in which the generation of a "J"-shaped mechanical behavior in a two-component system is possible. The results from the combination of an elastic inner layer coupled to a stiff outer layer (tensile element) are presented below. In this case, the inner layer is latex and the outer layer is suture, either wrapped polydioxannone (PDO), or stiched VICRYL™ (90:10 PLGA). Figure 14A-B shows a scaffold made from VICRYL™ sutured around the outer circumference of a latex tube. Suture was applied while the latex tube was expanded to a larger diameter. The latex tube was photographed at its resting diameter which is why the suture, applied at a larger diameter is forming loops around the circumference of the latex tube. Scale bar is 0.5 cm. A) axial view B) lateral view.

### Methods

Thin-walled latex tubing (Primeline Industries) with an inner diameter of 3.175 mm (D₁) was stretched onto a mandrel with an outer diameter of 8.0 mm (D₂) leading to a 151% increase in circumferential length. At the new, larger circumference, PDO suture (1.0 metric, Ethicon) was hand-wound in a spiral fashion down the length of the latex tube. The PDO suture was fixed in place by application of a thin layer of liquid latex (Environmental Technologies, Inc.) on top of the suture.

Following curing at room temperature and standard pressure (atmospheric pressure), the composite was removed from the mandrel, at which time the diameter returned to the initial diameter (D₁). The composite was then tested according to standard practices on an MTS Bionix tensile testing system (MTS, Inc.). Briefly, the tube was mounted in an *ad hoc* restraint and strain was applied at a rate of 5 mm/sec until failure occurred.

The same thin-walled latex tubing (D₁) was stretched onto a mandrel of larger diameter (D₂) leading to a 151% increase in circumferential length. At the new circumference, Vicryl suture material (1.5 metric, Ethicon) was hand-sutured around the circumference of the tube in a spiral fashion not penetrating more than half of the tube wall thickness. No adhesive coating was required. Testing was carried out to failure as previously described.

### Results

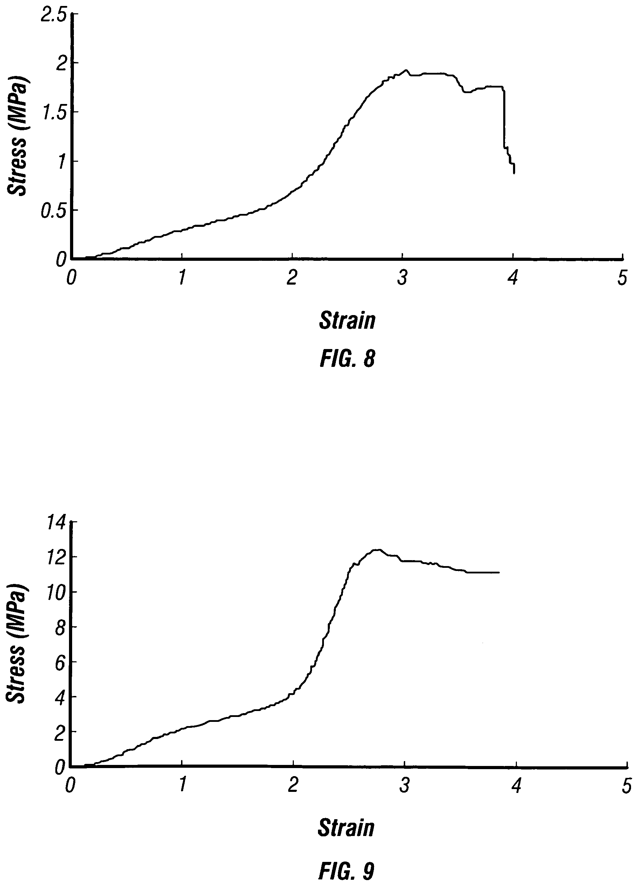

Respective tensile loading of these test specimens resulted in a "J"-shaped curve characterized by an initial low modulus (stiffness) region followed by a sharp upswing to a modulus of not less than one order-of-magnitude increase from the initial modulus. Figure 8 shows the resulting behavior of the latex/PDO architecture. Calculations of the initial and final modulus are 0.3 MPa and 2 MPa, respectively. Figure 9 shows the resulting behavior of the latex/Vicryl architecture. Moduli for this specimen were calculated to be 2 MPa and 20 MPa for the respective initial and final regions of the curve. Figure 10 demonstrates the respective stress/strain behaviors of PDO and Vicryl, which have respective elastic moduli of 3 GPa and 9GPa-18GPa. Figure 11 demonstrates the stress/strain relationship of latex, which has an elastic modulus of 0.3 MPa-0.5 MPa.

These results illustrate the feasibility of using a two component system to generate "J"-shaped mechanical behavior with the key factor involving stretching the elastic component prior to depositing the tensile component. Other variations in layer deposition are possible. For example, one or more layers created by wrapping, casting, electrospinning, or any combination thereof.

Material selection is also open to a wide range of combinations based on available materials as long as one material is highly elastic with a low modulus and the other material is high modulus (minimum of an order-of-magnitude greater than the other material) and low elasticity. Possible selections for the materials are described herein.

With the selection of different materials, different prestrain values, and different layer thicknesses, a high degree of tenability is available to "J"-shaped mechanical behavior in a scaffold design.

### EXAMPLE 2

A combination of an elastic inner layer coupled to a stiff outer layer (tensile element) was also examined. The inner layer is electrospun polyurethane (PU) and the outer layer is electrospun Poly glycolic acid (PGA).

### Methods

10% PU in 1,1,1,3,3,3-Hexafluoro-2-propanol (HFIP) and 10% PGA in HFIP were the base solutions used in electrospinning. Approximately 2 milliliters of 10% PU was electrospun onto a 5 mm OD mandrel utilizing standard electrospinning procedures. Following completion, the PU tube was rolled off of the 5 mm OD mandrel and rolled onto an 8 mm OD mandrel. Use of a 5 mm OD and 8 mm OD mandrel equates to a 60% increase in circumferential length.

10% PGA was then electrospun onto the surface of the dilated PU tube until fully coated which equated to an overall volume of approximately 1 ml of the PGA solution. Following coating, the hybrid tube was removed while care was taken to minimize delamination.

Subsamples were taken from pure PU and PGA tubes were tested along with the laminate hybrid according to standard practices on an MTS Bionix tensile testing system (MTS, Inc.). Briefly, the tubes were mounted in an *ad hoc* restraint and strain was applied at a rate of 5 mm/sec until failure occurred.

### Results.

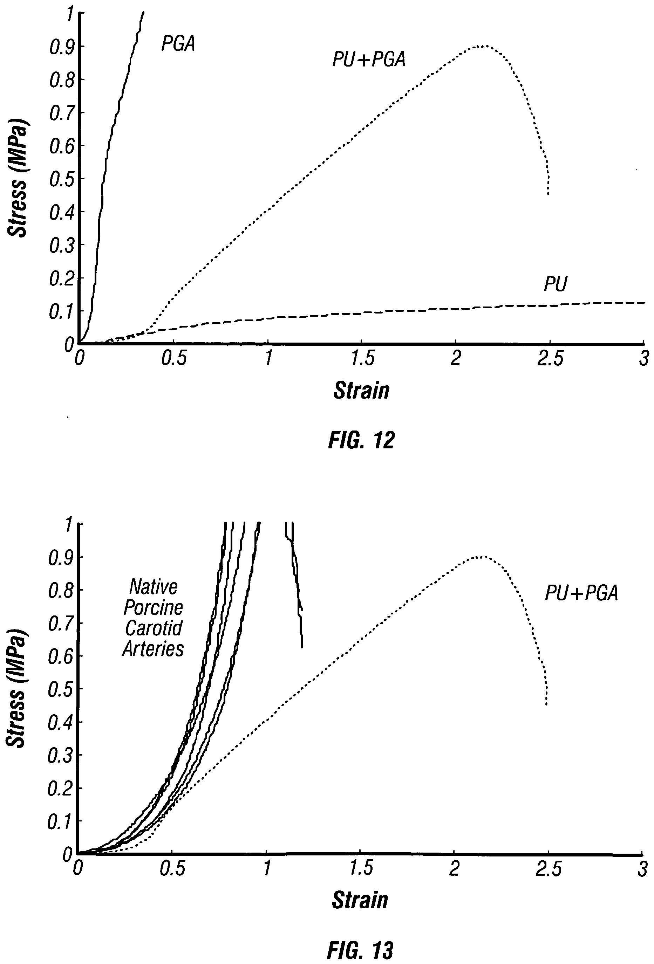

Figure 12 illustrates both the stress/strain behavior of tubes constructed from pure PGA and pure PU, as well as the resulting stress/strain behavior from a hybrid of both materials constructed as described above. Tensile loading of the hybrid resulted in a "J"-shaped curve characterized by an initial low modulus (stiffness) region followed by a sharp upswing to a modulus of approximately twice the value of the initial modulus (0.5 MPa versus 0.24 MPa).

Figure 13 shows the resulting stress/strain behaviors of the PU/PGA hybrid compared to native porcine carotid arteries.

These results support the feasibility of using at least a two component system to generate "J"-shaped mechanical behavior with an important factor involving stretching the elastic component prior to depositing the tensile component. Other iterations of the two component system will encompass variations in material selection and the deposition of additional layers. For example, both layers could be provided as a pre-formed layer, or formed by wrapping, casting, electrospinning, and any combination thereof.

### Example 3 - Scaffold formation using an expanding mandrel

Here, we describe a novel method that successfully recapitulates the complex stress/strain behavior of native vessels through a multi-component architectural modification. In addition, the method presents opportunities for the "tuning" of these complex biomechanical properties through a combination of material selection and variations in the formation processes. Tubular scaffolds made with Tecothane 1074 or Poly(L-lactide-co-*ε*-caprolactone, and Polyglycolic acid knitted mesh tubing generated native vessel characteristic stress/strain behavior with moduli of 0.5 MPa - 3.97 MPa and burst pressures averaging 1676 mm-Hg.

10% Polyurethane (PU: Tecothane 1074, Lubrizol, Inc.) and 12% Poly(L-lactide-co-*ε-*caprolactone) (PLCL: Lakeshore Biomaterials) were maintained as stock solutions in 1,1,1,3,3,3-Hexafluoro-2-propanol (HFIP: Sigma). 12 cm length tubes of these materials (4 mm-6 mm internal diameter, ~4-5 ml of stock solution) were formed through electrospinning as described elsewhere (Dahl 2007 *supra*). Electrospinning parameters for PU and PLCL are shown in Table 3.

**Table 3**

| | Mandrel speed (rpm) | Voltage (keV) | Infusion Rate (ml/hr) | Mandrel/ Needle Distance (cm) | Transverse Motion frequency (Hz) |
|---|---|---|---|---|---|
| PU | 5633 | 14 | 15 | 11 | 1.2 |
| PLCL | 5633 | 15 | 15 | 15.5 | 1.2 |

Following electrospinning, a custom mandrel is inserted into the tubes. The custom mandrel consists of multiple sections that are driven apart by end wedges while maintaining a circular cross section (exemplified in Fig. 5). In this fashion, the polymer tubes can be driven to larger internal/ external diameters for brief periods of time. For example, the mandrel allows the internal diameter (ID) of the tubes to be increased up to 160% for a tube with a 6 mm ID, or up to 250% for a tube with a 4 mm ID. After increasing the ID, the mandrel can be returned to its original setting to allow the tubes to recoil elastically to their original diameters.

6mm ID Tubes of either PU or PLCL were expanded to 140% of their original diameter (new diameter) following insertion into 8 mm ID polyglycolic acid (PGA) knitted mesh tubes (Concordia). At this new diameter, the PU or PLCL tubes were tightly bound by the knitted mesh PGA tubes. At this point, an additional thin layer (~1 ml polymer solution) of PU or PLCL was electrospun on top of the mesh and tube in such a fashion as to allow adhesion sandwiching of the mesh in-between the respective layers of synthetic (either PU or PLCL). The PU or PLCL tube/PGA mesh composite was allowed to return to the original diameter of the PU or PLCL tube originally utilized. Contraction of the underlying tube will entrain the mesh tube causing a corrugated (kinked) uniform surface feature. An illustration of this "expanding mandrel" process is presented in Fig 4E.

### Scaffold formation.

Fig 15A-B illustrates the gross appearance typical of scaffolds constructed using the expanding mandrel technique. Corrugations running the length of this PU/PGA scaffold are visible at lower magnification A), and at larger magnification B). The scale of the scaffold is ~12 cm.

Figure 16A-B shows a 5x cross sectional view of a PU/PGA scaffold and further illustrates the circumferentially uniform corrugations formed as a result of the expanding mandrel technique. The scale bar shown in Fig. 15A is 700 µm. In Fig. 16A, the PGA is not present, but the formation process remains the same as that conducted in Fig. 16B where PGA mesh is present. In both images, the corrugations can be seen as a result of the formation process with the corrugations being enhanced in Fig. 16B as a result of the presence of the mesh. The lesser degree of corrugation in Fig. 16A is due to the the additional layer of PU applied after expansion of the PU tube. The wall thickness and length of the scaffolds was typically on the order of 700 µm and 12 cm, respectively.

### Example 4 - Mechanical Testing

### Burst pressure Testing.

A burst testing apparatus, fabricated in-house, consisted of a high pressure syringe pump (Cole-Parmer), a stainless steel 20ml syringe (Cole-Parmer), and a calibrated 100 psi max liquid/gas pressure gage (Omega). The system was controlled using Labview v8.5 and a compact field point (National Instruments). In order to ensure no leaking during the testing, the inner lumen of the tubular scaffold was lined with a cylindrical 5mm ID standard latex balloon (Unique Industries, Inc.). Liquid volume was delivered to the scaffold at a stead rate of 1 ml/min until failure occurred. The maxima immediately preceding mechanical failure is the reported burst pressure value.

Circumferential testing (ring test): The scaffold was then tested according to standard practices on a MTS Bionix tensile testing system (MTS, Inc.). Briefly, the scaffold was mounted in an *ad hoc* restraint and displacement was applied at a rate of 5 mm/sec until failure occurred. The resulting raw force/ displacement data were converted to stress/ strain plots through careful micrometer measurement of the dimensions (thickness, starting length, width) of the tested scaffolds.

### Results.

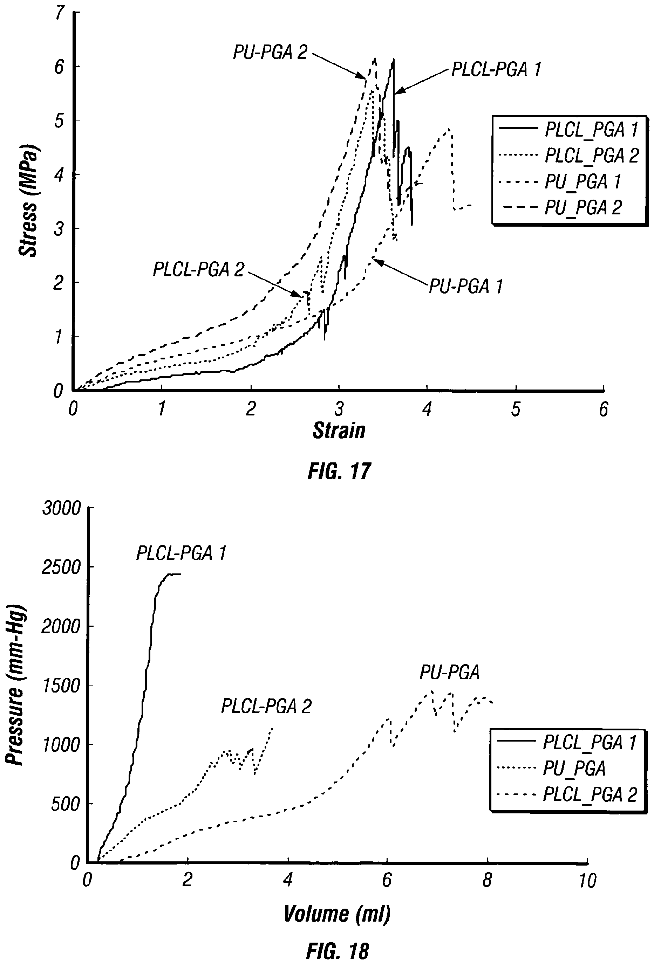

Tubular scaffolds that were tested consistently yielded stress/ strain behavior commensurate with a two-component system (Fig 17). All scaffolds demonstrate a mechanical behavior consisting of an initial low stiffness behavior (E= 0.5±0.24 MPa) that gives rise to a high stiffness zone (E= 3.97±1.6 MPa) at a transitional strain of 374±229% prior to mechanical failure. Fig 18 illustrates the results from burst pressure tests of tubular scaffolds. Overall burst pressures were 1676±676 mm-Hg. Summary data are presented in Table 4.

**Table 4**

| | Intial Modulus (MPa) | N | Final Modulus (MPa) | N | Inflection Pt. (%) | N | Burst Pressure (mm-Hg) | N |
|---|---|---|---|---|---|---|---|---|
| PU/PGA | 0.58±.23 | 5 | 3.4±1.5 | 5 | 410±265 | 5 | 1463 | 1 |
| PLCL/PGA | 0.3±0.08 | 2 | 5.5±0.707 | 2 | 280±94 | 2 | 1782±919 | 2 |
| Combined | 0.5±0.24 | 7 | 3.97±1.6 | 7 | 374±229 | 7 | 1676±676 | 3 |

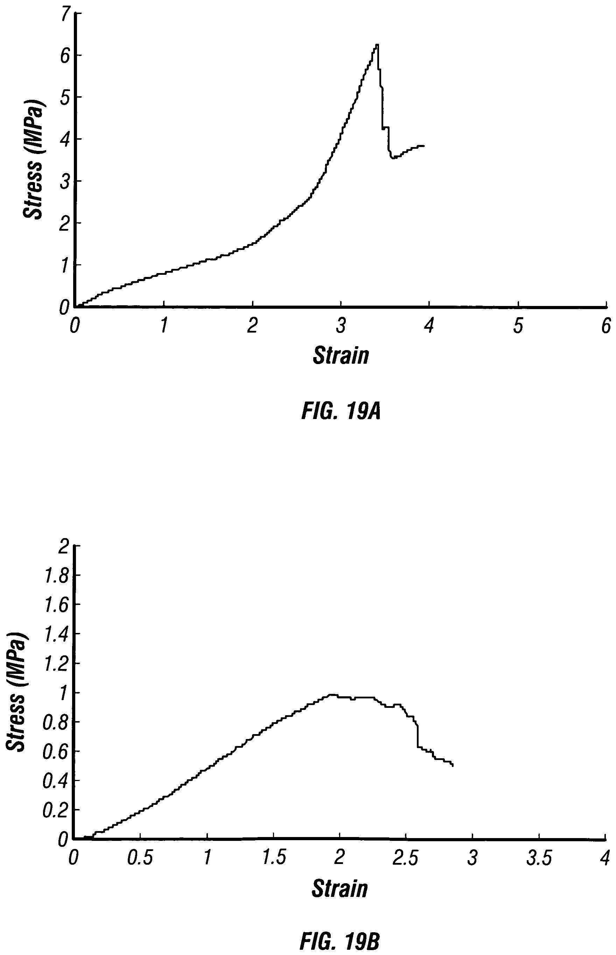

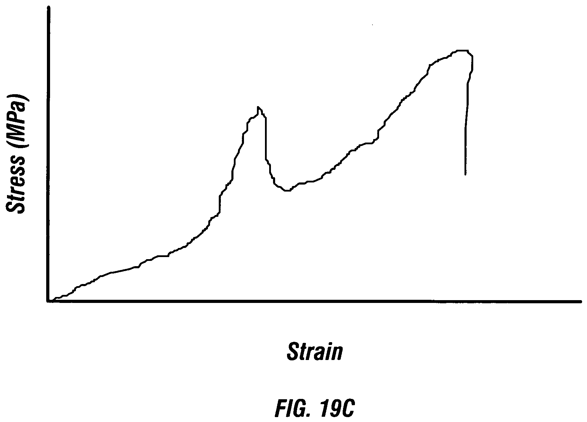

**Scaffold tunability:** The tubular scaffolds are multi-component systems with various degrees of freedom related to formation parameters such as the final diameter of the expanding mandrel utilized, PGA mesh stretch, and electrospun layer thickness. Fig. 19 demonstrates some of the variability in overall mechanical properties that is possible varying elements of the construction of these scaffolds. This indicates that the mechanical properties of the tubular scaffolds are tunable. Figure 19A depicts favorable mechanics where the failure of a PGA mesh tube coincides with the failure of a synthetic electrospun tube.

Figure 19B depicts the failure of an electrospun elastic tube prior to engagement of a reinforcing PGA mesh tube. In this case, the thin layer of PU or PLCL applied over the second tubular element when fitted over the first tubular element on the expanded mandrel is electrospun at a mandrel/needle distance of about one-half the normal distance, *i.e.,* about 5 cm to about 7 cm for PU and about 6 cm to about 8 cm for PLCL. The closer proximity of the needle means that the time the PU/PLCL solution is exposed to air as it travels from the needle to the surface of the second tubular element decreases, which results in a greater amount of solvent coming into contact with the second tubular element and the underlying first tubular element, as compared to electrospinning at the normal distance. The increased contact of solvent causes melting of the first tubular element, which makes the first tubular element more brittle.

Figure 19C depicts a hypothetical engagement and failure of a PGA mesh tube before the inner electrospun tube fails.

### Example 5 - Cell interaction with electrospun PLCL or PU

Glass coverslips were coated with a thin layer of electrospun PLCL or PU followed by coating with extracellular matrix proteins. For fibronectin coating, scaffolds were soaked overnight at 4C in 5ug/ml human fibronectin I (Chemicon FC010) in PBS. For low concentration collagen coating, scaffolds were soaked 1hr at RT in 50ug/ml rat tail collagen I (BD 354236) in 0.1% acetic acid, followed by a brief wash with PBS. Low concentration collagen scaffolds were air dried prior to seeding. High concentration collagen scaffolds were prepared by applying a thin layer of 3mg/ml rat tail collagen I (BD 354236), then exposing the scaffolds to ammonia vapor in a closed chamber for 3 minutes. High concentration collagen scaffolds were then washed briefly with water, followed by an overnight wash in PBS. Lastly, for MATRIGEL™ coating, scaffolds were covered with a thin layer of MATRIGEL™ solution (BD 356234) and incubated at 37C for 30 minutes to allow for protein polymerization.

Prior to seeding, all scaffolds were attached to the bottom of a 6-well cell culture dish with fibrin glue (Quixil). Human aortic endothelial cells (Cascade Biologicis, C-006) were resuspended in 250uL growth media and seeded directly onto scaffold surfaces at a density of 40,000 cells per cm². Seeded scaffolds were incubated at 37C, 5% CO₂ for 3 hours to allow for ample cell attachment. Wells were then filled with 3ml Media 200 (Cascade Biologics, M-200) supplemented with LSGS kit components (Cascade Biologics, S-003). Seeded scaffolds were cultured for 14 days with media changes occurring on every third day.

Seeded scaffolds were fixed in 4% paraformaldehyde in PBS overnight at 4C. Cells were stained with 2ug/ml CD31 (Dako M0823) primary antibody, followed by 2ug/ml Alexa488 goat antimouse IgG1 secondary antibody. Lastly, nuclei were stained with 3uM DAPI (Invitrogen).

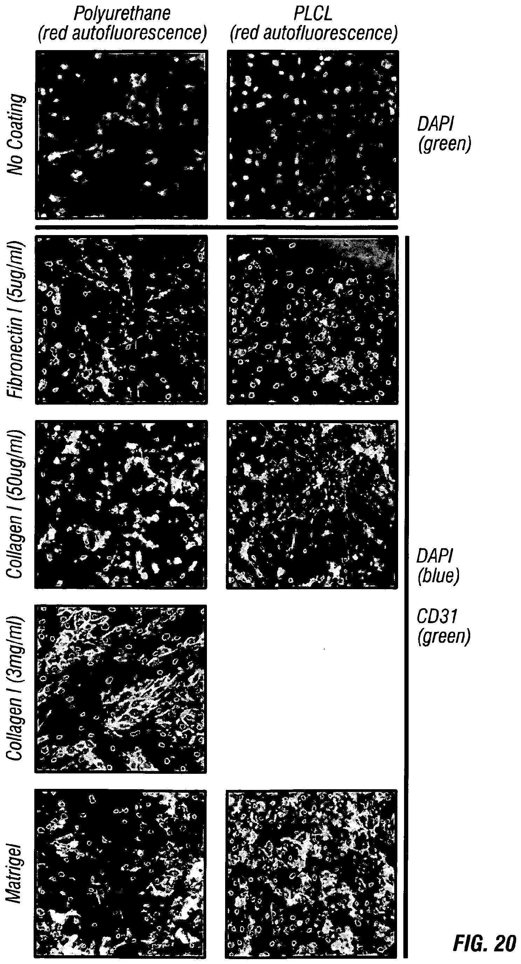

Figure 20 illustrates in static culture, the cellular attachment and spreading of cells on the two synthetics (PU/ PLCL) used as the inner structure in the tubular scaffolds. The histochemistry of electrospun synthetic polymers used in this study following treatment with cell attachment enhancing proteins: fibronectin, collagen, and MATRIGEL™ is shown. Without any coating PLCL retains more cells than PU. Of the three coatings: fibronectin, collagen 1, and MATRIGEL™, the MATRIGEL™ and Collagen 1 (dose dependent response) appeared to retain the highest number of cells. Furthermore, in the case of collagen 1 and MATRIGEL™ coatings, there was strong staining for CD31 where confluency was evident.

### Example 6 - Cell seeding and Bioreactor conditioning

Two tubular scaffolds were constructed as previously described from PLCL and PGA mesh having respective lengths of 6 cm and 10 cm, referred to as short and long, respectively.

**Cell Seeding:** Primary human aortic endothelial cells (HAEC; Cascade Biologics, C-006) were maintained in Medium 200 (Cascade Biologics, M-200) supplemented with 2% fetal bovine serum, 1ug/ml hydrocortisone, 10ng/ml hEGF, 3ng/ml bFGF, 10ug/ml heparin, and IX concentration of Gentamicin/Amphotericin B solution (Cascade Biologics, S-003). For seeding scaffolds, cells at passages 5-10 were trypsinized with 0.05% Trypsin-EDTA (Gibco, 25300) and resuspended in supplemented M-200 at 12x10⁶ per ml. Cell suspensions were injected into the vascular bioreactor through the distal port with ample volume to cover all luminal surfaces. After sealing all tubing, bioreactors were transferred to a roller bottle apparatus and rotated at 0.2rpm for 2 hrs at 37C. Following this step, bioreactor chambers were connected aseptically to the flow circuit described below.

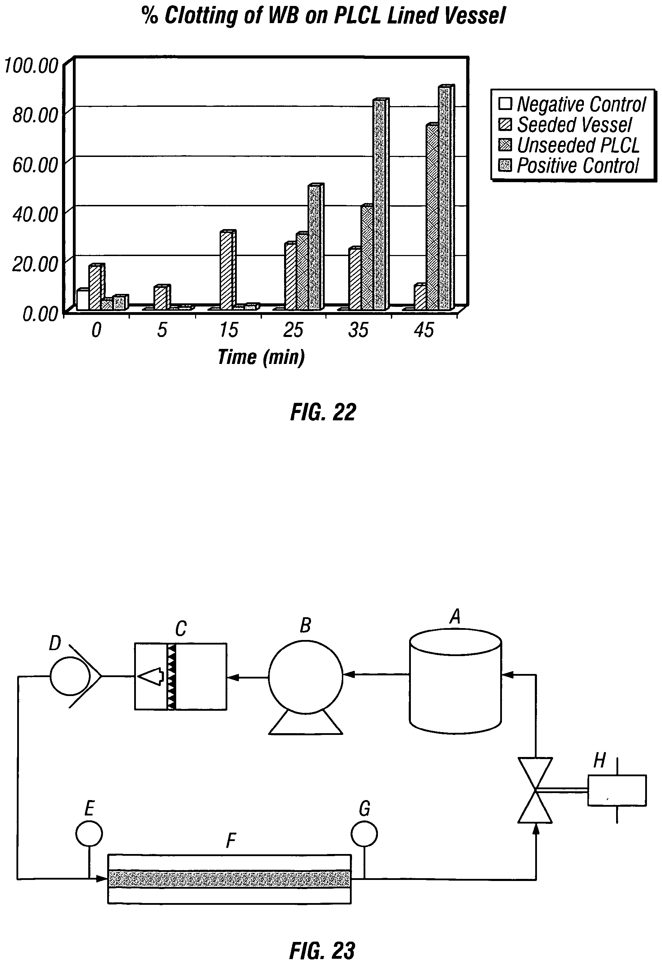

**Bioreactor conditioning:** As illustrated in Figure 23, a bioreactor system was fabricated in-house with a custom designed control system capable of imparting pulsatile flow. Flow from a reservoir (A) passes through a peristaltic pump (B) and into a pulse dampener (C) with a one-way check valve (D) to restrict retrograde flow. A pressure transducer (E) anterior to the bioreactor chamber where the scaffold is held (F) is followed by a posterior pressure transducer (G) and on into a pinch valve (H) prior to return to the reservoir. (Not pictured: computer control via compact field point)

Conditioning occurred over the course of 8 days based on a protocol (Table 5) designed to ease the seeded construct into physiological pulsatility and shear, thus maximizing the opportunity for cell attachment and integration.

**Table 5**

| | **ml/min** | **Shear (dyne/cm²)** | **t(hr)** | **Flow** |
|---|---|---|---|---|
| **Step 1** | 67.4 | 3.85 | 24 | Steady |
| **Step 2** | 82.3 | 4.7 | 24 | Steady |
| **Step 3** | 95.4 | 5.45 | 12 | Pulsatile |
| **Step 4** | 107.7 | 6.15 | 12 | Pulsatile |
| **Step 5** | 133.9 | 7.65 | 12 | Pulsatile |
| **Step 6** | 146.2 | 8.35 | 12 | Pulsatile |
| **Step 7** | 170.5 | 9.75 | 12 | Pulsatile |
| **Step 8** | 197 | 11.25 | 12 | Pulsatile |
| **Step 9** | 234.6 | 13.4 | 90 | Pulsatile |

Following the 8-day conditioning protocol, a series of cellular assays were utilized to assess the cell interaction with the construct.

### Example 7 - Conditioned scaffold cellular assays

**Live/Dead Staining (Invitrogen, L3224):** A single representative piece from the distal and proximal section of each vessel was reserved for fluorescent staining. The construct section was washed in an excess of DPBS. DPBS was removed and replaced with 2.5 ml of prepared stain. (10 ml DPBS, 20 µl calcein AM (green), 5 µl ethidium homodimer-1 (red). Following a 10-minute incubation the scaffold sections were visualized using the inverted fluorescent microscope. The pieces maintained a significant degree of curvature that made visualization quite difficult. Round cover slips were placed in the wells on top of the construct sections to help with flattening the pieces.

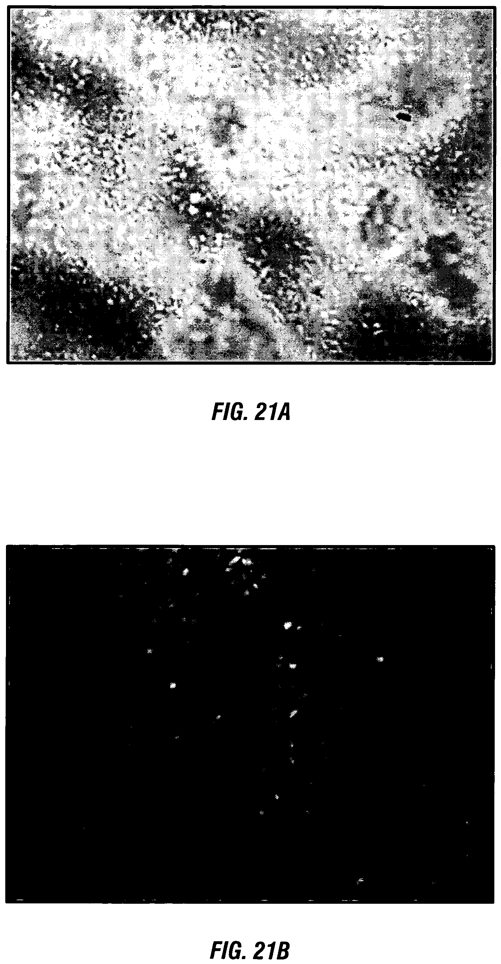

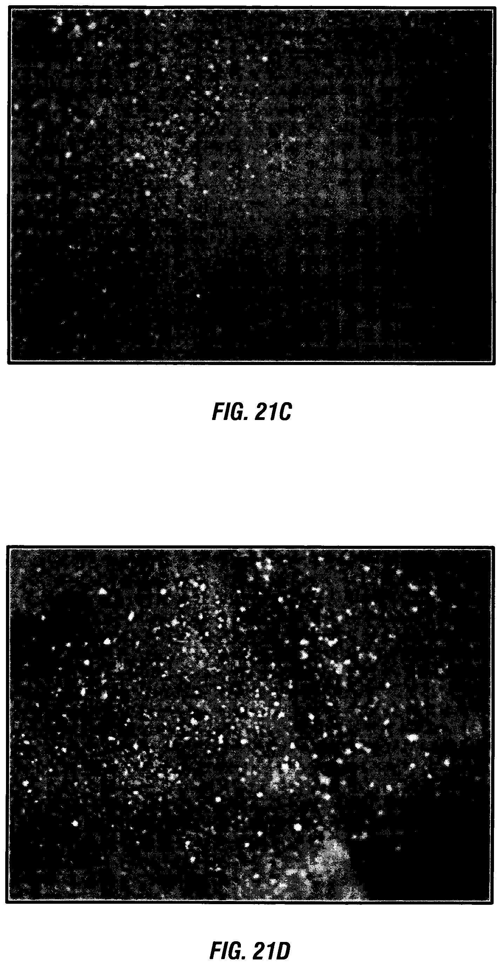

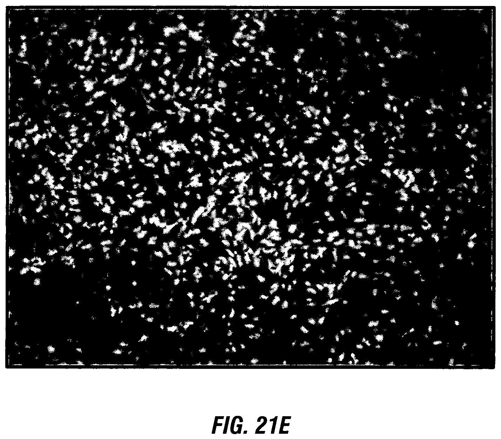

Figure 21 demonstrates that at the seeding density utilized in the experiment, cells were largely confluent with few indications of active cell death (A-short proximal; B-short proximal; C-long proximal; D-long distal; E-short distal). In the long segment samples, cells are rounded with no clear formation of an intact endothelium. Short segment samples show cells that cells have spread out on the scaffold and are clearly making cell-cell connections suggestive of a rudimentary endothelium.

The live/dead staining of proximal and distal segments (with respect to flow entry and exit) from the long and short PLCL/PGA mesh vascular tubular scaffolds following cell seeding and conditioning in a bioreactor is shown. Any non-viable cells are highlighted in red.

**Whole Blood Clotting Assay:** 4.25 ml of ACD whole blood was activated by the addition of 425 µl calcium chloride (0.1M). 10 µl aliquots of the well-mixed activated blood were placed on control or scaffold surfaces and incubated for varying lengths of time. At determined time points 300 µl of distilled water is added which lyses RBS not incorporated into a clot. Absorbance of resultant water/ hemoglobin solution is read which is inversely proportional to the amount of clotting. A glass cover slip served as positive control surface for clotting and a CoStar Low Binding 6 well plate served as the negative control surface.

Figure 22 shows clotting development as a function of time for seeded graft scaffolds compared with controls. Whole blood clotting on seeded, conditioned scaffold segments, positive and negative controls, as well as unseeded/unconditioned scaffold material, is shown. The positive control developments near-maximal clotting (85%) at 35 minutes and not much increase at 45 minutes. The unseeded control sees a rise from 40% clotting to maximal clotting (∼75%) between the 35 and 45 minute time points. The negative control shows trace clotting at the beginning of the experiment time point, but consistently demonstrates no clotting for all remaining timepoints. Lastly, the seeded graft shows maximal clotting (∼30%) at the 15 minute time point, but decreased to ∼10% clotting by 45 minutes.

**eNOS Detection:** eNOS production is indicative of a healthy, intact endothelium. Cellular associated eNOS was quantified using the R&D Systems eNOS ELISA system according to the manufacturer's protocol. Scaffold pieces, 4 pieces from each construct (2 distal and 2 proximal) were placed in microcentrifuge tubes with 150 µl of cell lysis buffer. These lysates were then frozen at -80 C until assayed. Upon thaw the lysate was centrifuged to remove cellular debris and 100 µl from each sample was available for assay.

Table 6 shows the results of eNOS production from seeded and conditioned graft tubular scaffolds. The detection of eNOS production in segments isolated from both the long and short seeded, conditioned tubular scaffolds is shown. eNOS is normalized to surface area of the graft. The short graft had large quantities of eNOS (∼500 pg eNOS/ 0.25 cm2) detected in both samples. The short scaffold had less than 62.5 pg eNOS/ 0.25 cm2 detected in each sample, placing the short graph below the threshold for positive eNOS reporting.

**Table 6**

| **Graft** | **Sample** | **eNOS Detected** | **pg eNOS/ .25 cm2** | **eNOS average** |
|---|---|---|---|---|
| **Short** | 1 | Yes | 492.4 | 495.9 |
| | 2 | Yes | 499.3 | |
| **Long** | 1 | No | < 62.5 | < 62.5 |
| | 2 | No | < 62.5 | |

**Metabolic analysis:** Each scaffold had 900mls of media for its 8-day incubation/ conditioning. The overall change detected in glucose and lactate for the short and long scaffold were comparable although glucose usage of was slightly less for the long scaffold compared with the short scaffold (0.07 g/L and .008 g/L respectively), with a lactate production of 0.053 g/L for each graft. Ammonia production was slightly higher for the long graft when compared with the short graft (0.880 mmol/L versus 0.783 mmol/L, respectively).

Table 7 shows the metabolic analysis of spent bioreactor media. Media reserved from each bioreactor was analyzed on Nova BioProfile 400 and the results were compared to fresh media control.

**Table 7**

| | **Bioreactor Media Vol** | **Change over Media Control** | | | | | |
|---|---|---|---|---|---|---|---|
| **Graft** | | **pH** | **Glucose g/L** | **Lactate g/L** | **Glutamine mmol/L** | **Glutamate mmol/L** | **NH4+ mmol/L** |
| Short | 900 | -0.211 | -0.083 | 0.053 | -1.037 | 0.057 | 0.783 |
| Long | 900 | -0.099 | -0.070 | 0.053 | -1.213 | 0.070 | 0.880 |

Examples 1-7 illustrate the feasibility of using a multi-component system to generate "J"-shaped mechanical behavior reminiscent of native vessels where the elastic component (PU or PLCL) is stretched prior to depositing the tensile component (PGA mesh tube). This technique provides a corrugated/kinked structure that will function in a similar fashion to that seen in vessels (albeit at a larger scale).

Mechanical testing of the tubular scaffolds demonstrated an order-of-magnitude difference between modulus 1 and modulus 2 commonly seen in native vessels (see Fig 1). Moreover, the choice of PGA as the tensile material and PLCL or PU as the elastic material was made in order to accurately match values seen in native vessels (Table 1). In fact, the technique for providing or forming the tubular scaffolds can be applied to many different material choices, synthetic or natural as long as one material is highly elastic with low modulus, and the other material is high modulus (min. order-of-magnitude greater than the other material) and low elasticity.

The average inflection point location (i.e. the strain at which the transition from modulus 1 to modulus 2 occurs) was ∼374% strain units. Typically, native vessels see this transition closer to ∼100% stain units (Table 1). The explanation for this value relates to the resting diameter and properties of the knitted PGA mesh tube utilized in the experiments and correspondingly the ability to increase the diameter of the expanding mandrel. For example, in order to shift the inflection point in a tubular scaffold to lower values, it must be understood how much a knitted tube will expand past its resting diameter before the fibers begin to experience loading. With the ability to tune the expansion properties of the knitted mesh prior to loading and its internal resting diameter, one can choose at what strain the mesh engages and consequently bind the inner elastic tube at this value.

PGA meshes can bear a significant amount of loading. In fact, this is demonstrated by the observation that an average physiologically-relevant burst pressure of 1676 mm-Hg can be observed. The method, however, is not limited to meshes. As mentioned above, different materials can be utilized, but different techniques for applying the tensile outer layer (as well as the inner elastic layer) can also be addressed. For example, future iterations might encompass layers being wrapped, cast, electrospun, or any combination thereof.

Cell seeding and bioreactor conditioning experiments provided insight into how the tubular scaffolds will perform *in vivo*. As shown in Figure 20, standard treatments for enhancing cell attachment such as precoating the scaffold with collagen 1, MATRIGEL™, or Fibronectin show marked improvement in both cell:cell and cell:biomaterial interaction with synthetic materials. Other methods for enhancing cell attachment include, but are not limited to, chemical modifications of the bulk synthetic polymers utilized in this study.

The live/dead assay of conditioned scaffolds showed little presence of non-viable cells. In fact, both the long graft and the short graft appeared to have the same coverage in cell density. However, on the long graft there was a clear difference in cell morphology. Although clotting results which are lumped in Figure 22 clearly show a benefit to the presence of cells on the graft, eNOS (Table 6) was lacking in the long scaffold. It is known that eNOS production is indicative of a healthy, intact endothelium, and although the cells are clearly present on the long graft, they are not spreading and forming connections with neighboring cells in the same fashion as that seen in the short scaffold. Given that these two scaffolds are of the same materials and were produced in the same fashion, this suggests that the conditioning of these scaffolds was somehow different. In fact, both grafts were given the same conditioning protocol, but one possible explanation is that geometrical considerations may have led to differences in flow within the different scaffolds. Given cell sensitivity to hydrodynamic factors, it may be that turbulent flow conditions led to the rounded morphology of the cells seen in the long scaffold and their consequent lack of eNOS expression. Furthermore, the discrepancy seen in eNOS expression might be indicated in the metabolic data (Table 7) that shows a slight variation in glucose consumption between the long and short scaffolds (long consumes slightly less) during the course of the 8 day conditioning protocol. This finding, along with the lack of eNOS production by the long graft is possibly indicative of a less active phenotype of cells in the long graft.

A novel structural technique is described herein for the formation of a multi-component tubular scaffold with the ability to mimic native vessel complex mechanics. The flexibility of the both the technique and material selection allows for fairly precise tuning and hence precise matching of vascular properties. In the future, *in vivo* animal experiments to assess the long term benefits of minimizing or removing compliance mismatch in the vascular graft milieu may be performed.

### Example 8 - Retention of seeded cells on a scaffold

The retention of cells seeded on a TEBV after *in vivo* implantation may be assessed in a modified version of Example 26 of Flugelman U.S. Published Patent Application No. 20070190037.

Tissue engineered blood vessels (TEBVs) of the present invention are prepared by seeding tissue engineered scaffolds on the luminal side with endothelial cells and on the adventitial side with smooth muscle cells.

Rabbits are anesthetized and then intubated. The monitoring system during the experiment includes blood pressure measurement, pulse oxymetry, and ECG. Heparin is injected intravenously for systemic anticoagulation following exposure and preparation of TEBVs for graft implantation. Blood samples are regularly taken during the procedure (e.g., every 30 minutes) to assess the efficacy of heparinization by measuring partial thromboplastin time (PTT).

The TEBVs are then implanted bilaterally end to side in carotid and femoral arteries. Patency of the TEBVs is assessed 30 minutes following exposure of the implanted TEBVs to blood flow and prior to harvesting by direct palpation, flow measurements using a Doppler flow meter (Transonic Animal Research Flowmeter, NY, USA) and by performing selective angiography.

The femoral and carotid implanted TEBVs are harvested two hours following implantation. The cellular retention on the interior surfaces of the harvested TEBVs is analyzed by fluorescence microscopy.

### Example 9 - In vivo Arterio-Venous Shunt (A-V shunt)

The *in vivo* effectiveness of the TEBVs of the present invention may be test in a modified version of the "*In Vivo* Rabbit Arterio-Venous Shunt Thrombosis Model" as described in Corte et al. U.S. Patent No. 7,459,564.

Rabbits of an appropriate weight are anesthetized. A saline-filled TEBV of the present invention is connected between the femoral arterial and the femoral venous cannulae. Blood will flow from the femoral artery via the TEBV, which acts as the aterio-venous shunt (AV-shunt), into the femoral vein. The patency of the TEBV can be assessed *in vivo* using this model using various techniques known in the art. For example, the presence of blood flow through the graft without significant stenosis and the absence of clogging are assessed. Ultrasound techniques may be used to observe the implanted TEBV. The ability of the TEBV to recover from needle puncturing is also used to test patency. At the end of the study, the animals are sacrificed and the implanted TEBVs are removed for further examination, such as the number of cells on the TEBV, and the extent to which the seeded cells have begun regenerative processes while *in vivo.* In addition, the mechanical properties of the TEBV are assessed and compared to the mechanical properties of a native blood vessel graft and/or the TEBV prior to implantation.

The same type of AV-shunt model may be used to test the patency of a TEBV used for an inter-positional blood vessel graft.

## Claims

1. A tissue engineering scaffold having a mechanical response to stress and strain substantially similar to that of a response by a native blood vessel, the scaffold comprising (a) a first tubular element comprising an elastomeric element, an exterior surface and an interior luminal surface; and (b) a second tubular element comprising a tensile corrugated element, an exterior surface and an interior luminal surface in contact with the exterior surface of the first tubular element, wherein the elastomeric element comprises an elastomeric component with a first elastic modulus and the tensile element comprises a tensile component with a second elastic modulus that is greater than the first elastic modulus, and wherein:
(1) the mechanical response of said tissue engineering scaffold to stress and strain is **characterized by** a J-shaped stress/strain curve; and/or
(2) the tissue engineering scaffold has at least one of:
(i) a circumferential tube elastic modulus 2 of about 3.0 MPa to about 6.0 MPa;
(ii) a circumferential modulus transition of about 0.57 MPa to about 1.12 MPa; and
(iii) a circumferential tube elastic modulus 1 of about 0.1 MPa to about 0.5 MPa.

2. A method of making a tissue engineering (TE) scaffold comprising the steps of:
(a) providing a first tubular element comprising an elastomeric element, an exterior surface, an interior luminal surface, and a first diameter;
(b) dilating the first tubular element to a second diameter;
(c) providing a second tubular element comprising a tensile corrugated element, an exterior surface and an interior luminal surface, on the surface of the dilated first tubular element of step (b);
(d) bonding the dilated first tubular element of step (b) and the second tubular element; and
(e) decreasing the second diameter of the first tubular element to the first diameter of step (a) to form the TE scaffold,
wherein the elastomeric element comprises an elastomeric component with a first elastic modulus and the tensile element comprises a tensile component with a second elastic modulus that is greater than the first elastic modulus.

3. The method of claim 2 wherein the providing step of (a) or the providing step of (c) comprises electrospinning on a mandrel.

4. The method of claim 2 wherein the providing step of (c) comprises placing a pre-formed second tubular element over the dilated first tubular element of step (b).

5. The tissue engineering scaffold of claim 1 or method of claim 2 wherein the corrugated second tubular element comprises a fibrous network in which the fiber direction is oriented circumferentially.

6. The tissue engineering scaffold or method of claim 1, 2 or 5 wherein the axis of the corrugations is parallel to the axial direction of the scaffold.

7. The tissue engineering scaffold of claim 1 or method of claim 2 wherein the second elastic modulus is greater than the first elastic modulus by at least one order of magnitude.

8. The tissue engineering scaffold of claim 1 or method of claim 2 wherein the elastomeric element comprises a natural elastomeric component and/or a synthetic elastomeric component.

9. The tissue engineering scaffold or method of claim 8 wherein the natural elastomeric component is selected from the group consisting of elastin, resilin, abductin, and silk.

10. The tissue engineering scaffold or method of claim 8 wherein the synthetic elastomeric component is selected from the group consisting of latex, a polyurethane (PU), polycaprolactone (PCL), poly-L-lactide acid (PLLA), polydiaxanone (PDO), poly(L-lactide-co-caprolactone) (PLCL), and poly(etherurethane urea) (PEUU).

11. The tissue engineering scaffold of claim 1 or method of claim 2 wherein the tensile element comprises a natural tensile component and/or a synthetic tensile component.

12. The tissue engineering scaffold or method of claim 11 wherein the natural tensile component is selected from the group consisting of collagen, cellulose, silk, and keratin.

13. The tissue engineering scaffold or method of claim 11 wherein the synthetic tensile component is selected from the group consisting of nylon, Dacron® (polyethylene terephthalate (PET)) Goretex® (polytetrafluoroethylene), polyester, polyglycolic acid (PGA), poly-lactic-co-glycolic acid (PLGA), and poly(etherurethane urea) (PEUU).

14. The tissue engineering scaffold of claim 1, which has at least one of the following:
(i) a pore gradient where the pore diameter gradually decreases from about 100 microns at the exterior surface of the second tubular element to about 5 to about 15 microns at the interior surface of the first tubular element;
(ii) a circumferential tube toughness of about 0.45 MJ/m3 to about 1.0 MJ/m3;
(iii) an axial tube toughness of about 0.1 MJ/m3 to about 0.5 MJ/m3;
(iv) a tangent delta of about 0.05 to about 0.3; and
(v) a storage modulus of about 400 MPa to about 0.12 MPa.

## Patentansprüche

1. Gewebebearbeitungsgerüst, das eine mechanische Reaktion auf Belastung und Beanspruchung aufweist, die im Wesentlichen jener einer Reaktion eines nativen Blutgefäßes ähnelt, wobei das Gerüst (a) ein erstes röhrenförmiges Element umfassend ein Elastomerelement, eine äußere Oberfläche und eine luminale innere Oberfläche; und (b) ein zweites röhrenförmiges Element umfassend ein gerilltes Zugelement, eine äußere Oberfläche und eine luminale innere Oberfläche in Kontakt mit der äußeren Oberfläche des ersten röhrenförmigen Elements umfasst, wobei das Elastomerelement eine Elastomerkomponente mit einem ersten Elastizitätsmodul umfasst und das Zugelement eine Zugkomponente mit einem zweiten Elastizitätsmodul umfasst, der größer ist als der erste Elastizitätsmodul, und wobei:
(1) die mechanische Reaktion des Gewebebearbeitungsgerüsts auf Belastung und Beanspruchung durch eine J-förmige Belastungs-/Beanspruchungskurve gekennzeichnet ist; und/oder
(2) das Gewebebearbeitungsgerüst zumindest eines der Folgenden aufweist:
(i) einen Umfangsrohr-Elastizitätsmodul 2 von etwa 3,0 MPa bis etwa 6,0 MPa;
(ii) einen Umfangsmodulübergang von etwa 0,57 MPa bis etwa 1,12 MPa; und
(iii) einen Umfangsrohr-Elastizitätsmodul 1 von etwa 0,1 MPa bis etwa 0,5 MPa.

2. Verfahren zur Herstellung eines Gewebebearbeitungs- (TE-) Gerüsts, das die folgenden Schritte umfasst:
(a) Bereitstellen eines ersten röhrenförmigen Elements umfassend ein Elastomerelement, eine äußere Oberfläche, eine luminale innere Oberfläche und einen ersten Durchmesser;
(b) Erweitern des ersten röhrenförmigen Elements auf einen zweiten Durchmesser;
(c) Bereitstellen eines zweiten röhrenförmigen Elements umfassend ein gerilltes Zugelement, eine äußere Oberfläche und eine luminale innere Oberfläche auf der Oberfläche des erweiterten ersten röhrenförmigen Elements aus Schritt (b);
(d) Bonden des erweiterten ersten röhrenförmigen Elements aus Schritt (b) und des zweiten röhrenförmigen Elements;
und
(e) Verkleinern des zweiten Durchmessers des ersten röhrenförmigen Elements auf den ersten Durchmesser aus Schritt (a), um das TE-Gerüst zu bilden,
wobei das Elastomerelement eine Elastomerkomponente mit einem ersten Elastizitätsmodul umfasst und das Zugelement eine Zugkomponente mit einem zweiten Elastizitätsmodul umfasst, der größer ist als der erste Elastizitätsmodul.

3. Verfahren nach Anspruch 2, wobei der Bereitstellungsschritt aus (a) oder der Bereitstellungsschritt aus (c) das Elektrospinnen auf einer Spindel umfasst.

4. Verfahren nach Anspruch 2, wobei der Bereitstellungsschritt aus (c) das Positionieren eines vorgeformten zweiten röhrenförmigen Elements über dem erweiterten ersten röhrenförmigen Element aus Schritt (b) umfasst.

5. Gewebebearbeitungsgerüst nach Anspruch 1 oder Verfahren nach Anspruch 2, wobei das gerillte zweite röhrenförmige Element ein faserartiges Netz umfasst, in dem die Faserrichtung in Richtung des Umfangs ausgerichtet ist.

6. Gewebebearbeitungsgerüst oder Verfahren nach Anspruch 1, 2 oder 5, wobei die Achse der Rillen parallel zur Achsenrichtung des Gerüsts ist.

7. Gewebebearbeitungsgerüst nach Anspruch 1 oder Verfahren nach Anspruch 2, wobei der zweite Elastizitätsmodul um zumindest eine Größenordnung größer ist als der erste Elastizitätsmodul.

8. Gewebebearbeitungsgerüst nach Anspruch 1 oder Verfahren nach Anspruch 2, wobei das Elastomerelement eine natürliche Elastomerkomponente und/oder eine synthetische Elastomerkomponente umfasst.

9. Gewebebearbeitungsgerüst oder Verfahren nach Anspruch 8, wobei die natürliche Elastomerkomponente aus der Gruppe bestehend aus Elastin, Resilin, Abductin und Seide ausgewählt ist.

10. Gewebebearbeitungsgerüst oder Verfahren nach Anspruch 8, wobei die synthetische Elastomerkomponente aus der Gruppe bestehend aus Latex, einem Polyurethan (PU), Polycaprolacton (PCL), Poly-L-Milchsäure (PLLA), Polydiaxanon (PDO), Poly(L-lactid-co-caprolacton) (PLCL) und Poly(etherurethanharnstoff) (PEUU) ausgewählt ist.

11. Gewebebearbeitungsgerüst nach Anspruch 1 oder Verfahren nach Anspruch 2, wobei das Zugelement eine natürliche Zugkomponente und/oder eine synthetische Zugkomponente umfasst.

12. Gewebebearbeitungsgerüst oder Verfahren nach Anspruch 11, wobei die natürliche Zugkomponente aus der Gruppe bestehend aus Collagen, Cellulose, Seide und Keratin ausgewählt ist.

13. Gewebebearbeitungsgerüst oder Verfahren nach Anspruch 11, wobei die synthetische Zugkomponente aus der Gruppe bestehend aus Nylon, Dacron® (Polyethylenterephthalat (PET)), Goretex® (Polytetrafluorethylen), Polyester, Polyglykolsäure (PGA), Poly-milch-co-glykolsäure (PLGA) und Poly(etherurethanharnstoff) (PEUU) ausgewählt ist.

14. Gewebebearbeitungsgerüst nach Anspruch 1, das zumindest eines der Folgenden aufweist:
(i) einen Porengradienten, wobei der Porendurchmesser schrittweise von etwa 100 µm an der äußeren Oberfläche des zweiten röhrenförmigen Elements bis auf etwa 5 bis etwa 15 µm an der inneren Oberfläche des ersten röhrenförmigen Elements abnimmt;
(ii) eine Röhrenumfangszähigkeit von etwa 0,45 MJ/m³ bis etwa 1,0 MJ/m³;
(iii) eine Röhrenachsenzähigkeit von etwa 0,1 MJ/m³ bis etwa 0,5 MJ/m³;
(iv) ein Tangensdelta von etwa 0,05 bis etwa 0,3; und
(v) einen Speichermodul von etwa 400 MPa bis etwa 0,12 MPa.

## Revendications

1. Échafaudage de génie tissulaire présentant une réponse mécanique aux contraintes et aux déformations sensiblement similaire à celle d'une réponse par un vaisseau sanguin natif, l'échafaudage comprenant (a) un premier élément tubulaire comprenant un élément élastomère, une surface extérieure et une surface luminale intérieure ; et (b) un second élément tubulaire comprenant un élément ondulé extensible, une surface extérieure et une surface luminale intérieure en contact avec la surface extérieure du premier élément tubulaire, dans lequel l'élément élastomère comprend un composant élastomère ayant un premier module d'élasticité et l'élément extensible comprend un composant extensible ayant un second module d'élasticité qui est supérieur au premier module d'élasticité, et dans lequel :
(1) la réponse mécanique dudit échafaudage de génie tissulaire aux contraintes et aux déformations est **caractérisée par** une courbe contrainte/déformation en forme de J ; et/ou
(2) l'échafaudage de génie tissulaire présente au moins l'un de :
(i) un module d'élasticité de tube circonférentiel 2 d'environ 3,0 MPa à environ 6,0 MPa ;
(ii) une transition de module circonférentiel d'environ 0,57 MPa à environ 1,12 MPa ; et
(iii) un module d'élasticité de tube circonférentiel 1 d'environ 0,1 MPa à environ 0,5 MPa.

2. Procédé de fabrication d'un échafaudage de génie tissulaire (GT) comprenant les étapes suivantes :
(a) fourniture d'un premier élément tubulaire comprenant un élément élastomère, une surface extérieure, une surface luminale intérieure et un premier diamètre ;
(b) dilatation du premier élément tubulaire à un second diamètre ;
(c) fourniture d'un second élément tubulaire comprenant un élément ondulé extensible, une surface extérieure et une surface luminale intérieure, sur la surface du premier élément tubulaire dilaté de l'étape (b) ;
(d) collage du premier élément tubulaire dilaté de l'étape (b) et du second élément tubulaire ; et
(e) réduction du second diamètre du premier élément tubulaire jusqu'au premier diamètre de l'étape (a) pour former l'échafaudage de GT,
dans lequel l'élément élastomère comprend un composant élastomère ayant un premier module d'élasticité et l'élément extensible comprend un composant extensible ayant un second module d'élasticité qui est supérieur au premier module d'élasticité.

3. Procédé selon la revendication 2 dans lequel l'étape de fourniture de (a) ou l'étape de fourniture de (c) comprend le filage électrostatique sur un mandrin.

4. Procédé selon la revendication 2 dans lequel l'étape de fourniture de (c) comprend la mise en place d'un second élément tubulaire préformé sur le premier élément tubulaire dilaté de l'étape (b).

5. Échafaudage de génie tissulaire selon la revendication 1 ou procédé selon la revendication 2 dans lequel le second élément tubulaire ondulé comprend un réseau fibreux dans lequel les fibres sont orientées dans direction circonférentielle.

6. Échafaudage de génie tissulaire ou procédé selon la revendication 1, 2 ou 5 dans lequel l'axe des ondulations est parallèle à la direction axiale de l'échafaudage.

7. Échafaudage de génie tissulaire selon la revendication 1 ou procédé selon la revendication 2 dans lequel le second module d'élasticité est supérieur au premier module d'élasticité d'au moins un ordre de grandeur.

8. Échafaudage de génie tissulaire selon la revendication 1 ou procédé selon la revendication 2 dans lequel l'élément élastomère comprend un composant élastomère naturel et/ou un composant élastomère synthétique.

9. Échafaudage de génie tissulaire ou procédé selon la revendication 8 dans lequel le composant élastomère naturel est choisi dans le groupe constitué de l'élastine, de la résiline, de l'abductine et de la soie.

10. Échafaudage de génie tissulaire ou procédé selon la revendication 8 dans lequel le composant élastomère synthétique est choisi dans le groupe constitué du latex, d'un polyuréthane (PU), de la polycaprolactone (PCL), de l'acide de poly-L-lactide (PLLA), de la polydiaxanone (PDO), de la poly(L-lactide-co-caprolactone) (PLCL) et de la poly(étheruréthane urée) (PEUU).

11. Échafaudage de génie tissulaire selon la revendication 1 ou procédé selon la revendication 2 dans lequel l'élément extensible comprend un composant extensible naturel et/ou un composant extensible synthétique.

12. Échafaudage de génie tissulaire ou procédé selon la revendication 11 dans lequel le composant extensible naturel est choisi dans le groupe constitué du collagène, de la cellulose, de la soie et de la kératine.

13. Échafaudage de génie tissulaire ou procédé selon la revendication 11 dans lequel le composant extensible synthétique est choisi dans le groupe constitué du nylon, du Dacron® (téréphtalate de polyéthylène (PET)), du Goretex® (polytétrafluoroéthylène), du polyester, de l'acide polyglycolique (PGA), de l'acide poly-lactique-co-glycolique (PLGA) et de la poly(étheruréthane urée) (PEUU).

14. Échafaudage de génie tissulaire selon la revendication 1, qui présente au moins l'une des caractéristiques suivantes :
(i) un gradient de pore où le diamètre de pore diminue progressivement d'environ 100 microns au niveau de la surface extérieure du second élément tubulaire à environ 5 à environ 15 microns au niveau de la surface intérieure du premier élément tubulaire ;
(ii) une résistance de tube circonférentiel d'environ 0,45 MJ/m3 à environ 1,0 MJ/m3 ;
(iii) une résistance de tube axial d'environ 0,1 MJ/m3 à environ 0,5 MJ/m3 ;
(iv) une tangente delta d'environ 0,05 à environ 0,3 ; et
(v) un module de conservation d'environ 400 MPa à environ 0,12 MPa.
